(19) Europäisches Patentamt
European Patent Office
Office européen des brevets

(11) EP 1 811 989 B1

(12) EUROPÄISCHE PATENTSCHRIFT

(45) Veröffentlichungstag und Bekanntmachung des Hinweises auf die Patenterteilung:
**21.04.2010 Patentblatt 2010/16**

(21) Anmeldenummer: **05801751.8**

(22) Anmeldetag: **02.11.2005**

(51) Int Cl.:
***A61K 31/353*** *(2006.01)* ***A61K 8/49*** *(2006.01)*
***A61Q 19/00*** *(2006.01)*

(86) Internationale Anmeldenummer:
**PCT/EP2005/011687**

(87) Internationale Veröffentlichungsnummer:
**WO 2006/053637 (26.05.2006 Gazette 2006/21)**

# (54) ZUBEREITUNG ENTHALTEND OXIDIERTE FLAVONOID-DERIVATE

PREPARATION CONTAINING OXIDIZED FLAVONOID DERIVATIVES

PREPARATION CONTENANT DES DERIVES DE FLAVONOIDE OXYDES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **19.11.2004 DE 102004055932**

(43) Veröffentlichungstag der Anmeldung:
**01.08.2007 Patentblatt 2007/31**

(73) Patentinhaber: **Merck Patent GmbH**
**64293 Darmstadt (DE)**

(72) Erfinder:
- **CAROLA, Christophe**
  **69120 Heidelberg (DE)**
- **HUBER, Sylvia**
  **64646 Heppenheim (DE)**
- **BUCHHOLZ, Herwig**
  **60599 Frankfurt (DE)**

(56) Entgegenhaltungen:
**DE-A- 3 601 414**

- **IGARASHI, K. ET AL.: "An Oxidation Product of Quercetin Catalyzed by a Crude Enzyme Preperation from Red Clover (Trifolium pratense L.); Its Isolation and Identification" AGRIC. BIOL. CHEM, Bd. 54, Nr. 8, 1990, Seiten 2143-2144, XP008058315 in der Anmeldung erwähnt**
- **IGARASHI, K. ET AL.: "Aerial Oxidation Products of Quercetin in Acidic Solution" AGRIC. BIOL. CHEM., Bd. 42, Nr. 8, 1978, Seiten 1617-1719, XP008058316**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 811 989 B1

## Beschreibung

Die Erfindung betrifft neue Zubereitungen, insbesondere kosmetische und/oder pharmazeutische Zubereitungen oder Nahrungsergänzungsmittel, enthaltend mindestens ein oxidiertes Flavonoid-Derivat der Formel I, neue oxidierte Flavonoid-Derivate sowie neue Verwendungen der oxidierten Flavonoid-Derivate.

Die menschliche Haut unterliegt Alterungsprozessen, die teilweise auf intrinsische Prozesse (chrono-ageing) und teilweise auf exogene Faktoren (environmental, z.B. photo-ageing) zurückzuführen sind. Zusätzlich können vorübergehende oder auch andauernde Veränderungen des Hautbildes auftreten wie z. B. Akne, tätige oder trockene Haut, Keratosen, Rosaceae, wie empfindliche, entzündliche, erythematöse, allergische oder autoimmunreaktive Reaktion wie Dermatosen und Photodermatosen.

Zu den exogenen Faktoren zählen insbesondere das Sonnenlicht oder künstliche Strahlenquellen mit vergleichbarem Spektrum sowie Verbindungen, die durch Strahlung entstehen können, wie undefinierte reaktive Photoprodukte, die auch radikalisch oder ionisch sein können. Zu diesen Faktoren zählen auch Zigarettenrauch und die darin enthaltenen reaktiven Verbindungen wie Ozon, freie Radikale, beispielsweise das Hydroxylradikal, Sigulettsauerstoff und andere reaktive Sauerstoff- und Stickstoffverbindungen, die die natürliche Physiologie oder Morphologie der Haut stören.

Hautalterung geht mit einer Verringerung der Schichtdicken der beiden aufeinander liegenden Hautschichten Epidermis und Dermis einher und es wird angenommen, dass dies zumindest teilweise für die Bildung von Falten in der alternden Haut verantwortlich ist. Während die oben liegende Epidermis der Haut vor allem Widerstandsfähigkeit verleiht und die Hauptbarriere ausbildet, verleiht die darunter liegende Dermis der Haut Stärke, Elastizität und Dicke.
Die Epidermis besteht hauptsächlich aus Keratinozyten, die in vier unterschiedliche Differenzierungsstadien unterteilt werden können. Die epidermale Differenzierung ist sehr wichtig für die Ausbildung der essenziellen Hautfunktionen, nämlich als Schutzbarriere gegenüber der Umwelt sowie zur Verhinderung von Wasserverlust vom Körper. In der letzten Stufe der epidermale Differenzierung werden die verhornten Zellhüllen (cornified cell envelope) ausgebildet. Dabei kommt es unter Einwirkung der Transglutaminase zu einer Quervernetzung der Proteine Loricrin, kleiner Prolin-reicher Proteine und Involucrin. Aktivierung der Transglutaminase ist daher auch ein vielversprechenden Ansatz zur Verbesserung der Hautstruktur und zur Bekämpfung der Hautalterung (anti-aging).

Zum Schutz der Haut gegenüber Lichtexposition können kosmetische und/oder pharmazeutische Produkte verwendet werden, die UV-Filter enthalten. Vorteilhaft sind dabei insbesondere Wirkstoffe, die neben einem UV-Schutz auch antioxidativ wirken und die Haut somit sowohl durch Verminderung der Lichtexposition als auch durch Inaktivierung von durch Strahlenexposition induzierten oder in sonstiger Weise gebildeten freien Radikalen entgegenwirken.

Bekannte Antioxidantien sind Flavonoide, die vielfach auch in dermatologischen Zubereitungen Verwendung finden. Nachteilhaft können die Flavonoide auf Grund ihrer geringen Wasserlöslichkeit jedoch in wässrige Formulierungen nur in vergleichsweise geringen Mengen in antioxidativ wirksame Zubereitungen eingearbeitet werden. So weist beispielsweise Quercetin (Cyanidanol, Cyanidenolon 1522, Meletin, Sophoretin, Ericin, 3,3',4',5,7-Pentahydroxyflavon), das häufig als besonders wirksames Antioxidantien genannt wird (z.B. C.A. Rice-Evans, N.J. Miller, G. Paganga, Trends in Plant Science 1997, 2(4), 152-159), eine Löslichkeit in Wasser von nur 0,04 g/l auf.

K. Lemanska, H. Szymusiak, B. Tyrakowska, R. Zielinski, A.E.M.F. Soffers, I.M.C.M. Rietjens; Free Radical Biology&Medicine 2001, 31(7), 869-881 untersuchen die pH-Abhängigkeit der antioxidativen Wirkung von Hydoxyflavonen. Über den gesamten pH-Bereich zeigt Quercetin die höchste Aktivität der untersuchten Strukturen.

DE 36 01 414 offenbart die Gewinnung antiinflammatorisch wirksamer Flavonoide ähnlich vorliegender Formel (I) aus Podophyllum, wobei $R^1$-$R^4$, $R^6$-$R^9$ Wasserstoff entsprechen und austelle von $OR^5$ eine Methylgruppe vorhanden ist.

Die Wirkung von Antioxidantien wird darauf zurückgeführt, dass sie leichter oxidierbar sind als die zu schützenden Substanzen. Sind Antioxidantien anwesend, werden diese bei oxidativem Stress an Stelle der zu schützenden Substanzen oxidiert, sodass die zu schützenden Substanzen nicht oxidiert und hierdurch geschützt werden. Jedoch werden die Antioxidantien in Folge ihrer Oxidation "verbraucht", wodurch sie der Zubereitung mit ihrer (Schutz)-Funktion als Antioxidantien entzogen werden sollten.

Aufgabe der Erfindung ist es, eine antioxidativ wirksame Zubereitung zur Verfügung zu stellen, enthaltend mindestens einen antioxidativ wirksamen Wirkstoff mit hoher antioxidativer Wirksamkeit, worin der Wirkstoff eine hinreichend gute Löslichkeit aufweist, dass dieser in der Zubereitung in für die jeweils gewünschte Wirkungsstärke erforderlicher Konzentration enthalten sein kann und/oder welche eine schützende Wirkung gegen UV-Strahlen aufweist und/oder einer Alterung der Haut entgegenwirkt.

Überraschenderweise wurde nun gefunden, dass die Verbindungen der allgemeinen Formel I, stark antioxidativ wirken, obwohl diese Oxidationsprodukte von Flavonoid-Derivaten sind und beispielsweise als Oxidationsprodukte von Quercetin angesehen werden können.

Auf Grund ihrer antioxidativen Wirkung sind die Verbindungen der allgemeinen Formel I hervorragend als Wirkstoffe für antioxidativ wirksame Zubereitungen geeignet, beispielsweise für solche, die der Hautalterung entgegenwirken. Gegenstand der vorliegenden Erfindung ist daher eine antioxidativ wirksame Zubereitung enthaltend mindestens eine Verbindung der allgemeinen Formel I

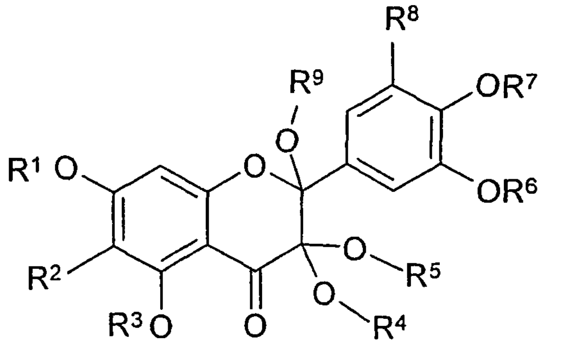

I

worin

| | |
|---|---|
| $R^1$, $R^3$, $R^4$, $R^5$, $R^6$, $R^7$, $R^9$ | jeweils unabhängig voneinander H oder Alkyl und |
| $R^2$, $R^8$, | jeweils unabhängig voneinander H, OH oder -O-Alkyl ist, |

sowie gegebenenfalls Träger- und/oder Hilfsstoffe.

Alkyl bedeutet jeweils geradkettiges oder verzweigtes $C_1$-$C_{10}$-Alkyl mit 1, 2, 3, 4, 5, 6, 7, 8, 9 oder 10 Kohlenstoffatomen und bedeutet daher bevorzugt Methyl, Ethyl, n-Propyl, Isopropyl, n-Butyl, sek.-Butyl, tert.-Butyl, Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3-oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, Heptyl, Octyl, Nonyl oder Decyl. Besonders bevorzugt ist $C_1$-$C_6$-Alkyl und hierbei insbesondere Butyl.

Nach einer vorteilhaften Ausführungsform der Erfindung enthält die Zubereitung zumindest eine Verbindung der Formel I, die dadurch gekennzeichnet ist, dass $R^1$, $R^3$, $R^4$, $R^5$, $R^6$, $R^7$, $R^9$ jeweils unabhängig voneinander H, verzweigtes oder gradkettiges $C_1$-$C_6$-Alkyl, vorzugsweise Methyl, Ethyl, n-Propyl, n-Butyl ist, und $R^2$ und $R^8$ unabhängig voneinander H oder OH ist. Bevorzugt ist in der in der erfindungsgemäßen Zubereitung enthaltenen Verbindung der Formel I $R^4 = R^5$.

Nach einer besonders vorteilhaften Ausführungsform der Erfindung enthält die Zubereitung zumindest eine Verbindung der Formel I, die dadurch gekennzeichnet ist, dass $R^1$ bis $R^8$ jeweils H ist und $R^9$ H oder $C_{1-6}$-Alkyl, insbesondere Methyl, Ethyl, n-Propyl oder n-Butyl ist.

Oxidierte Flavonoid-Derivate der Formel I, in denen $R^1$ bis $R^8$ jeweils H und $R^9$ Methyl oder Ethyl ist, sind bekannt aus Agric Biol. Chem. 54 (8), 2143-2144 (1990), wo sie aus sonnengetrocknetem roten Klee isoliert wurden. Die oxidierten Flavonoid-Derivate entsprechen oxidiertem Quercetin, wobei dieses anscheinend z.B. infolge seiner Aufarbeitung mit Methanol als Extraktionsmittel zu dem entsprechenden 2-O-Methylderivat umgesetzt wird.
Die Publikation untersucht den Abbau von einem ernährungsphysiologisch wertvollen Protein-Isolat in rotem Klee, der sich durch andauernde Sonneneinstrahlung ergibt. Die Autoren vermuten, dass die mit dem Proteinabbau einhergehende Schädigung der Aminosäurereste durch die gefundenen oxidierten Flavonoid-Derivate bewirkt wird. Somit lässt sich dieser Arbeit keinerlei Lehre entnehmen, dass die Verbindungen antioxidative Eigenschaften aufweisen und daher als Antioxidantien verwendet werden könnten. Im Gegenteil, die Vermutung, die Oxidationsprodukte von Quercetin trügen zum Proteinabbau bei, führt geradewegs weg von ihrer Verwendung als Antioxidantien. Denn Antioxidantien sollten derartige Schädigungen ja gerade verhindern.

Vorteilhaft weisen die Verbindungen der allgemeinen Formel I gegenüber den als Antioxidantien bekannten Flavonoiden, wie beispielsweise Quercetin, in polaren Lösungsmitteln, insbesondere in Wasser, eine deutlich höhere Löslichkeit auf und können daher in einfacher Weise und in großer Menge in die erfindungsgemäße Zubereitung eingearbeitet werden. Beispielhaft erwähnt sei hier die Löslichkeit der Verbindung 2-Butoxy-2-(3,4-dihydroxy-phenyl)-3,3,5,7-tetrahydroxy-chroman-4-on bei 25°C in Wasser (2,9 g/l) sowie in Ethanol (855 g/l). Die Löslichkeiten von Quercetin in Wasser bzw. in Ethanol liegen dagegen bei nur 0,04 g/l bzw. 15,8 g/l.

Weiterhin absorbieren die Verbindungen der Formel I in einem sehr breiten Bereich ultraviolette Strahlung. Die Verbindungen sind daher auch besonders geeignet zur Verwendung als UV-Filter.

Vorteile der erfindungsgemäßen Zusammensetzungen sind insbesondere ihre antioxidative Wirkung und die

gute Hautverträglichkeit. Zusätzlich sind bevorzugte der hier beschriebenen Verbindungen farblos oder nur schwach gefärbt und führen so nicht oder nur in geringer Weise zu Verfärbungen der Zubereitungen. Von Vorteil ist insbesondere das besondere Wirkprofil der erfindungsgemäß einzusetzenden Verbindungen, welches sich im DPPH-Assay in einer hohen Kapazität Radikale zu fangen ($EC_{50}$), einer zeitverzögerten Wirkung ($T_{EC50}$ > 120 min) und damit einer mittleren bis hohen antiradikalischen Effizienz (AE) äußert. Zudem vereinigen die Verbindungen nach Formel I im Molekül antioxidative Eigenschaften mit UV-Absorption im UV-A- und/oder -B-Bereich.

2,2-Diphenyl-1-picrylhydrazyl(DPPH)-Assay: 2,2-Diphenyl-1-picrylhydrazyl ist ein in Lösung stabiles freies Radikal. Das ungepaarte Elektron führt zu einer starken Absorptionsbande bei 515 nm, die Lösung ist dunkel-violett gefärbt. In Gegenwart eines Radikalfängers wird das Elektron gepaart, die Absorption verschwindet und die Entfärbung verläuft stöchiometrisch unter Berücksichtigung der aufgenommenen Elektronen. Gemessen wird die Extinktion im Photometer. Die antiradikalische Eigenschaft der zu testenden Substanz wird bestimmt, indem man die Konzentration ermittelt, bei der 50 % des eingesetzten 2,2-Diphenyl-1-picrylhydrazyls mit dem Radikalfänger reagiert haben. Ausgedrückt wird diese Konzentration als $EC_{50}$, ein Wert, der unter den gegebenen Messbedingungen als Substanzeigenschaft zu betrachten ist. Verglichen wird die untersuchte Substanz mit einem Standard (z.B. Tocopherol). Der $EC_{50}$-Wert ist dabei ein Maß für die Kapazität der jeweiligen Verbindung Radikale zu fangen. Je niedriger der $EC_{50}$-Wert ist, desto höher ist die Kapazität Radikale zu fangen. Im Sinne dieser Erfindung wird von einer großen oder hohen Kapazität Radikale zu fangen gesprochen, wenn der $EC_{50}$-Wert niedriger als der von Tocopherol.

Der $T_{EC50}$-Wert (gemessen in Minuten) ergibt sich aus der Zeit, in der der $EC_{50}$-Wert erreicht wird, und beschreibt somit die Geschwindigkeit, mit der diese Antioxidantien Radikale fangen. Im Sinne dieser Erfindungen gelten Antioxidantien, die diesen Wert innerhalb von weniger als 60 Minuten erreichen, als schnell, solche, die den $EC_{50}$-Wert erst nach mehr als 120 Minuten erreichen, als zeitverzögert wirkend.

Die antiradikalische Effizienz (AE) (beschrieben bei C. Sanchez-Moreno, J.A. Larrauri und F. Saura-Calixto in J. Sci. Food Agric. 1998, 76(2), 270-276.) ergibt sich aus den oben genannten Größen nach folgender Beziehung:

$$AE = \frac{1}{EC_{50} T_{EC50}}$$

Eine niedrige AE (x$10^{-3}$) liegt im Bereich bis etwa 10, von einer mittleren AE wird im Bereich von 10 bis 20 gesprochen und eine hohe AE liegt erfindungsgemäß bei Werten oberhalb 20 vor.

Ein Gegenstand der vorliegenden Erfindung ist daher auch die Verwendung der Verbindungen gemäß Formel I, wie oben angegeben, als Antioxidationsmittel mit lang anhaltender Wirkung bzw. zur Herstellung einer Zubereitung mit antioxidativen Eigenschaften.

Unter Zubereitung wird eine Formulierung verstanden, die die Verbindung der Formel I enthält und zur Anwendung am Mensch oder Tier bestimmt ist, beispielsweise durch Auftragen auf die Haut, zur oralen Einnahme, Inhalation, Infusion oder Injektion. Je nach Art der Zubereitung kann die Zubereitung neben einer Verbindung der Formel I Träger- und/oder Hilfsstoffe enthalten, sie kann aber auch ausschließlich aus der Verbindung der Formel I selbst bestehen, beispielsweise in Form eines Pulvers, das z.B. direkt oral eingenommen oder inhaliert werden kann. Neben den Verbindungen der Formel I kann die Zubereitung auch weitere Wirkstoffe enthalten.

Bei den Zubereitungen handelt es sich dabei üblicherweise um pharmazeutische und/oder kosmetische Zubereitungen, insbesondere um topisch anwendbare Zubereitungen, beispielsweise kosmetische oder dermatologische Formulierungen, oder um Nahrungsmittel bzw. Nahrungsergänzungsmittel. Die Zubereitungen enthalten einen für die jeweilige Art der Zubereitung geeigneten Träger und, je nach gewünschtem Eigenschaftsprofil, optional weitere geeignete Inhaltsstoffe.

Nach einer Ausführungsform ist die erfindungsgemäße Zubereitung dadurch gekennzeichnet, dass diese eine pharmazeutische Zubereitung ist. Hierbei mindestens eine Verbindung der Formel I zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Nach einer bevorzugten Ausführungsform ist die erfindungsgemäße Zubereitung dadurch gekennzeichnet, dass diese ein Arzneimittel ist.

Die Arzneimittel können in der Human- oder Veterinärmedizin verwendet werden. Als Trägerstoffe kommen organische oder anorganische Substanzen in Frage, die sich für die enterale (z.B. orale), parenterale oder topische Applikation eignen und mit den Verbindungen der Formel I nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung

Lösungen, vorzugsweise ölige oder wässrige Lösungen, ferner Suspensionen, Emulsionen oder Implantate, für die topische Anwendung Salben, Cremes oder Puder. Die Pflanzenextrakte können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder mehrere weitere Wirkstoffe enthalten, z.B. ein oder mehrere Vitamine.

Die Verbindungen der für Formel I werden in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt.

Die pharmazeutischen Formulierungen enthaltend mindestens eine Verbindung der Formel I können mit Hilfe von Techniken hergestellt werden, die dem Fachmann wohl bekannt sind.

Besonders bevorzugt ist eine Zubereitung, die dadurch gekennzeichnet ist, dass sie ein Hautbehandlungsmittel ist.

Ein Hautbehandlungsmittel ist eine kosmetische, dermatologische oder pharmazeutische Zubereitung, die sich zur topischen Anwendung eignet. Typischerweise enthält die Zubereitung dabei übliche, hautvertragliche und entsprechend des Verwendungszweckes getestete Träger und ggf. weitere Hilfsmittel und Wirkstoffe.

Besonders bevorzugt ist daher weiterhin eine Zubereitung, die dadurch gekennzeichnet, dass diese ein kosmetisches Mittel ist.

Die Verbindungen der Formel I erhöhen die Widerstandskraft der Haut gegenüber Umwelteinflüssen, wie beispielsweise Austrocknung, wirken der Hautalterung entgegen, führen zu einer Verbesserung der Hautstruktur, insbesondere zur Bildung von glatter Haut und sind antientzündlich wirksam. Gegenstand der Erfindung ist daher auch die Verwendung der Verbindungen der Formel I zur Herstellung einer pharmazeutischen und/oder kosmetischen Zubereitung zur Erhöhung der Widerstandskraft der Haut gegenüber Umwelteinflüssen, insbesondere Austrocknung, zur Vermeidung der Hautalterung, zur Verbesserung der Hautstruktur, insbesondere zur Bildung glatter Haut.

Die Verbindungen der Formel I wirken auch als Radikalfänger und damit oxidativem Stress entgegen. Weiterhin weisen Sie eine anti-allergische und anti-irritative Wirkung auf und können somit zur Behandlung oder vorbeugenden Behandlung von Allergien, Entzündungen und Irritationen, insbesondere der Haut, verwendet werden. Gegenstand der Erfindung ist daher auch die Verwendung der Verbindungen der Formel I zur Herstellung einer pharmazeutischen und/oder kosmetischen Zubereitung zum Schutz gegen oxidativen Stress sowie zur Bekämpfung von Allergien, Entzündungen und/oder Irritationen. Bevorzugt sind Zubereitungen zur topischen Anwendung auf der Haut.

Damit die Verbindungen der Formel I ihre positive Wirkung als Radikalfänger auf die Haut besonders gut entwickeln können, kann es bevorzugt sein die Verbindungen der Formel I in tiefere Hautschichten eindringen zu lassen. Soweit die Eindringtiefe in epidermale Schichten nicht ausreichend ist, kann diese durch geeignete Transportmittel, beispielsweise Liposomen, erhöht werden, die einen Transport der der Verbindung durch die äußeren Hautschichten ermöglichen. Schließlich ist auch ein systemischer Transport der Verbindungen der Formel I denkbar. Die Zubereitung wird dann beispielsweise so gestaltet, dass sie für eine orale Gabe geeignet ist.

Allgemein wirken die Substanzen der Formel I als Radikalfänger. Solche Radikale werden nicht nur durch Sonnenlicht erzeugt, sondern werden unter verschiedenen Bedingungen gebildet. Beispiele sind Anoxie, die den Elektronenfluß stromauf der Cytochromoxidasen blockiert und die Bildung von Superoxidradikalarionen bedingt; Entzündungen, die unter anderem mit der Bildung von Superoxidanionen durch die Membran-NADPH-Oxidase der Leukozyten einhergehen, die jedoch auch mit der Bildung (durch Disproportionierung in Gegenwart von Eisen (II)-ionen) der Hydroxyradikale und anderer reaktiver Spezies, die normalerweise beim Phänomen einer Phagocytose beteiligt sind, einhergehen; sowie Lipidautooxidation die im Allgemeinen durch ein Hydroxylradikal initiiert wird und lipidische Alkoxyradikale und Hydroperoxide liefert.

Aufgrund ihrer Wirkungen eignen sich die Verbindungen der Formel I auch zur Herstellung von Zubereitungen zur Immunprotektion und zum Schutz der DNA und RNA. Die erhaltenen Zubereitungen eignen sich insbesondere zum Schutz von DNA und RNA vor oxidativen Angriffen, vor Radikalen und vor Schädigung durch Strahlung, insbesondere UV-Strahlung. Soweit die Verbindung in Form von Zubereitungen zur Anwendung auf der Haut verwendet werden, ergibt sich gegenüber UV-Strahlung eine doppelte Schutzwirkung: durch die Absorption von UV-Strahlung, die deren Einwirkung auf die Haut verhindert, sowie durch die Wirkung als Radikalfänger, die den durch dennoch eindringende UV-Strahlung induzierten Radikalen entgegengewirkt.

Es wird vermutet, dass bevorzugte Verbindungen der Formel I auch als Enzymhemmer wirken. Sie hemmen vermutlich Histidindecarboxylase, Proteinkinasen, Elastase, Aldosereduktase sowie Hyaluronidase, und ermöglichen daher, die Unversehrtheit der Grundsubstanz vaskulärer Hüllen aufrecht zu erhalten. Ferner hemmen sie vermutlich

nicht spezifisch Katechol-O-methyltransferase, wodurch die Menge der verfügbaren Katecholamine und dadurch die Gefäßfestigkeit erhöht wird. Weiter hemmen sie AMP-Phosphodiesterase, wodurch die Substanzen ein Potential zur Hemmung der Thrombozytenaggregation aufweisen.

Aufgrund dieser Eigenschaften eignen sich die erfindungsgemäßen Zubereitungen allgemein zur Immunprotektion und zum Schutz der DNA und RNA. Insbesondere eignen sich die Zubereitungen dabei zum Schutz von DNA und RNA vor oxidativen Angriffen, vor Radikalen und vor Schädigung durch Strahlung, insbesondere UV-Strahlung. Ein weiterer Vorteil der erfindungsgemäßen Zubereitungen ist der Zellschutz, insbesondere der Schutz von Langerhans-Zellen vor Schäden durch die oben genannten Einflüsse. Alle diese Verwendungen bzw. die Verwendung der Verbindungen der Formel I zur Herstellung entsprechend einsetzbarer Zubereitungen sind ausdrücklich auch Gegenstand der vorliegenden Erfindung.

Insbesondere eignen sich bevorzugte erfindungsgemäße Zubereitungen auch zur Behandlung von Hautkrankheiten, die mit einer Störung der Keratinisierung verbunden sind, die die Differenzierung und Zellproliferation betrifft, insbesondere zur Behandlung der Akne vulgaris, Akne comedonicä, der polymorphen Akne, der Akne rosaceae, der nodulären Akne, der Akne conglobata, der altersbedingten Aknen, der als Nebenwirkung auftretenden Aknen, wie der Akne solaris, der medikamenten-bedingten Akne oder der Akne professionalis, zur Behandlung anderer Störungen der Keratinisierung, insbesondere der Ichtyosen, der ichtyosiformen Zustände, der Darrier-Krankheit, der Keratosis palmoplantaris, der Leukoplasien, der leukoplasiformen Zustände, der Haut- und Schleimhautflechten (Buccal) (Lichen), zur Behandlung anderer Hauterkrankungen, die mit einer Störung der Keratinisierung zusammenhängen und eine entzündliche und/oder immunoallergische Komponente haben und insbesondere aller Formen der Psoriasis, die die Haut, die Schleimhäute und die Finger und Zehennägel betreffen, und des psoriatischen Rheumas und der Hautatopien, wie Ekzemen oder der respiratorischen Atopie oder auch der Hypertrophie des Zahnfleisches, wobei die Verbindungen ferner bei einigen Entzündungen verwendet werden können, die nicht mit einer Störung der Keratinisierung zusammenhängen, zur Behandlung aller gutartigen oder bösartigen Wucherungen der Dermis oder Epidermis, die gegebenenfalls viralen Ursprungs sind, wie Verruca vulgaris. Veruca plana, Epidermodysplasia verruciformis, orale Papillomatose, Papillomatosis florida, und der Wucherungen, die durch UV-Strahlung hervorgerufen werden können, insbesondere des Epithelioma basocellulare und Epithelioma spinocellulare, zur Behandlung anderer Hautkrankheiten, wie der Dermatitis bullosa und der das Kollagen betreffenden Krankheiten, zur Behandlung bestimmter Augenkrankheiten, insbesondere der Hornhauterkrankungen, zur Behebung oder Bekämpfung der lichtbedingten und der mit dem Älterwerden zusammenhängenden Hautalterung, zur Verminderung der Pigmentierungen und der Keratosis actinica und zur Behandlung aller Krankheiten, die mit der normalen Alterung oder der lichtbedingten Alterung zusammenhängen, zur Vorbeugung vor oder der Heilung von Wunden/Narben der Atrophien der Epidermis und/oder Dermis, die durch lokal oder systemisch angewendete Corticosteroide hervorgerufen werden und aller sonstigen Arten der Hautatrophie, zur Vorbeugung vor oder Behandlung von Störungen der Wundheilung, zur Vermeidung oder Behebung von Schwangerschaftsstreifen oder auch zur Förderung der Wundheilung, zur Bekämpfung von Störungen der Talgproduktion, wie Hyperseborrhö bei Akne oder der einfachen Seborrhö, zur Bekämpfung von oder Vorbeugung von krebsartigen Zuständen oder vor präkanzerogenen Zuständen, insbesondere der promyelozytären Leukämien, zur Behandlung von Entzündungserkrankungen, wie Arthritis, zur Behandlung aller virusbedingten Erkrankungen der Haut oder anderer Bereiche des Körpers, zur Vorbeugung vor oder Behandlung der Alopecie, zur Behandlung von Hautkrankheiten oder Krankheiten anderer Körperbereiche mit einer immunologischen Komponente, zur Behandlung von Herz-/Kreislauf-Erkrankungen, wie Arteriosklerose oder Bluthochdruck, sowie des Insulin-unabhängigen Diabetes, zur Behandlung von Hautproblemen, die durch UV-Strahlung hervorgerufen werden.

Ein weiterer Gegenstand der vorliegenden Anmeldung ist daher eine Zubereitung zur topischen Anwendung umfassend

a) mindestens eine Verbindung der Formel I, wie oben beschrieben,
b) einen hautverträglichen Träger, und
c) optional einen oder mehrere weitere Wirkstoffe mit hautpflegender und/oder entzündungshemmender Wirkung.

In einer bevorzugten Ausführungsform der vorliegenden Erfindungen handelt es sich bei der Zubereitung daher um eine Zubereitung zum Schutz von Körperzellen gegen oxidativen Stress, insbesondere zur Verringerung der Hautalterung, dadurch gekennzeichnet, dass sie neben einer oder mehrerer Verbindungen nach Formel I vorzugsweise ein oder mehrere weitere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und

Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen topischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004). Derartige Antioxidantien werden mit Verbindungen der Formel I in solchen Zusammensetzungen überlicherweise in Verhältnissen im Bereich von 1000:1 bis 1:1000, bevorzugt in Mengen von 100:1 bis 1:100 eingesetzt.

Es kann erfindungsgemäß insbesondere bevorzugt sein, schnell wirkende Antioxidantien mit solchen mit langsamer oder zeitverzögerter Wirkung zu kombinieren. Dabei sind typische Gewichtsverhältnisse der schnell wirkenden Antioxidantien zu zeitverzögert wirkenden Antioxidantien im Bereich 10:1 bis 1:10, vorzugsweise im Bereich 10:1 bis 1:1 und für hautschützende Zubereitungen insbesondere bevorzugt im Bereich 5:1 bis 2:1. In anderen erfindungsgemäß ebenfalls bevorzugten Zubereitungen kann es im Sinne einer Wirkungsoptimierung allerdings von Vorteil sein, mehr zeitverzögert wirkende Antioxidantien als schnell wirkende Antioxidanten vorliegen. Typische Zusammensetzungen zeigen dann Gewichtsverhältnisse der schnell wirkenden Antioxidantien zu zeitverzögert wirkenden Antioxidantien im Bereich 1:1 bis 1:10, vorzugsweise im Bereich 1:2 bis 1:8.

Die schützende Wirkung gegen oxidativen Stress bzw. gegen die Einwirkung von Radikalen kann also weiter verbessert werden, wenn die Zubereitungen ein oder mehrere weitere/s Antioxidan/s/tien enthält/enthalten, wobei es dem Fachmann keinerlei Schwierigkeiten bereitet geeignet schnell oder zeitverzögert wirkende Antioxidantien auszuwählen.

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C und dessen Derivaten, DL-α-Tocopherol, Tocopherol-E-Acetat, Nicotinsäure, Pantothensäure und Biotin. Vitamine werden dabei mit Verbindungen der Formel I üblicherweise in Verhältnissen im Bereich von 1000:1 bis 1:1000, bevorzugt in Mengen von 100: 1 bis 1:100 eingesetzt.

Unter anderem auf Grund der antioxidativen Wirkung der Verbindungen der Formel I eignen sich eine diese auch zum Schutz menschlicher Haut bzw. zum Schutz von Körperzellen gegen oxidativen Stress, d.h. z.B. gegen Schädigungen durch Radikale, wie sie durch Sonneneinstrahlung erzeugt werden. Der Schutz gegenüber ultraviolette Strahlung durch die erfindungsgemäße Zubereitung kann durch Einarbeitung eines oder mehrerer weiterer UV-Filter/s noch verstärkt werden.

Zusätzlich haben solche bevorzugte Verbindungen Vorteile bei der Einarbeitung in die Zubereitungen:

- sind $R^1$, $R^3$, $R^4$, $R^5$, $R^6$, $R^7$ und/oder $R^9$ = H und/oder sind $R^2$ und/oder $R^8$ = OH verbessert dies die Wasserlöslichkeit der erfindungsgemäß einzusetzenden Verbindungen;
- sind $R^1$, $R^3$, $R^4$, $R^5$, $R^6$, $R^7$ und/oder $R^9$ geradkettige oder verzweigte $C_1$- bis $C_{10}$-Alkylgruppen, insbesondere eine langkettige Alkylgruppe, und/oder sind $R^2$ und/oder $R^8$ = H erhöht dies die Öllöslichkeit der Verbindungen;

d.h. über die geeignete Auswahl der Substituenten kann die Hydrophilie bzw. Lipophilie der erfindungsgemäßen Verbindungen gesteuert werden.

In ebenfalls bevorzugten Ausführungsformen der Erfindung können die Verbindungen der allgemeinen Formel I auch in über ihre Löslichkeit hinausgehender Menge in der Zubereitungs-Matrix enthalten sein. In diesem Fall liegen die Verbindungen vorzugsweise in feinteiliger Form in der Zubereitung dispergiert vor.

Erfindungsgemäß insbesondere bevorzugte Zubereitungen enthalten neben den Verbindungen der Formel I auch reine UV-Filter.

Gegenstand der Erfindung ist daher weiterhin eine Zubereitung enthaltend eine oder mehrere Verbindung/en der Formel I, die dadurch gekennzeichnet ist, dass sie weiterhin einen oder mehrere UV-Filter enthält.

Prinzipiell kommen alle UV-Filter für eine Kombination mit den erfindungsgemäßen Verbindungen der Formel I in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.

Benzylidenkampferderivate wie

- 3-(4'-Methylbenzyliden)-dl-kampfer (z.B. Eusolex® 6300),
- 3-Benzylidenkampfer (z.B. Mexoryl® SD),
- Polymere von N-{(2 und 4)-[(2-oxoborn-3-yliden)methyl]benzyl}-acrylamid (z.B. Mexoryl® SW),
- N,N,N-Trimethyl-4-(2-oxoborn-3-ylidenmethyl)anilinium methylsulfat (z.B. Mexoryl® SK) oder
- α-(2-Oxoborn-3-yliden)toluol-4-sulfonsäure (z.B. Mexoryl® SL),

Benzoyl- oder Dibenzoylmethane wie

- 1-(4-tert-Butylphenyl)-3-(4-methoxyphenyl)propan-1,3-dion (z.B. Eusolex® 9020) oder
- 4-Isopropyldibenzoylmethan (z.B. Eusolex® 8020),

Benzophenone wie

- 2-Hydroxy-4-methoxybenzophenon (z.B. Eusolex® 4360) oder
- 2-Hydroxy-4-methoxybenzophenon-5-sulfonsäure und ihr Natriumsalz (z.B. Uvinul® MS-40),

Methoxyzimtsäureester wie

- Methoxyzimtsäureoctylester (z.B. Eusolex® 2292),
- 4-Methoxyzimtsäureisopentylester, z.B. als Gemisch der Isomere (z.B. Neo Heliopan® E 1000),

Salicylatderivate wie

- 2-Ethylhexylsalicylat (z.B. Eusolex® OS),
- 4-Isopropylbenzylsalicylat (z.B. Megasol®) oder
- 3,3,5-Trimethylcyclohexylsalicylat (z.B. Eusolex® HMS),

4-Aminobenzoesäure und Derivate wie

- 4-Aminobenzoesäure,
- 4-(Dimethylamino)benzoesäure-2-ethylhexylester (z.B. Eusolex® 6007),
- ethoxylierter 4-Aminobenzoesäureethylester (z.B. Uvinul® P25),

Benzimidazolderivate wie

- 2-Phenylbenzimidazol-5-sulfonsäure sowie ihre Kalium-, Natrium- und Triethanolaminsalze (z.B. Eusolex® 232),
- 2,2'-(1,4-Phenylen)bis-(1H-benzimidazol-4,6-disulfonsäure, Mononatriumsalz) (CAS-Nr. 180 898-37-7),
- 2,2'-(1,4-Phenylen)bis-(1H-benzimidazol-5-sulfonsäure) sowie ihre Kalium-, Natrium- und Triethanolaminsalze,

und weitere Substanzen wie

- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX),
- 2,4,6-Trianilino-(p-carbo-2'-ethylhexyl-1'-oxi)-1,3,5-triazin (z.B. Uvinul® T 150),
- 2-(2H-Benzotriazol-2-yl)-4-methyl-6-(2-methyl-3-(1,3,3,3-tetramethyl-1-(trimethylsily/oxy)disiloxanyl)propyl)phenol

(z.B. Silatrizole®),

- 4,4'-[(6-[4-((1,1- Dimethylethyl) aminocarbonyl) phenylamino]- 1,3,5- triazin- 2,4- diyl) diimino] bis (benzoesäure- 2-ethylhexylester) (z.B. Uvasorb® HEB),
- α-(Trimethylsilyl)-ω-[trimethylsilyl)oxy]poly[oxy(dimethyl [und ca. 6% methyl[2-[p-[2,2-bis(ethoxycarbonyl]vinyl] phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1),
- 2,2'-Methylen-bis-(6-(2H-benzotriazol-2-yl)-4-(1,1,3,3-tetramethylbutyl)phenol) (CAS-Nr. 103 597-45-1),
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).

Die in der Liste aufgeführten Verbindungen sind nur als Beispiele aufzufassen. Selbstverständlich können auch andere UV-Filter verwendet werden.

Diese organischen UV-Filter werden in der Regel in einer Menge von 0,5 bis 10 Gew.-%, vorzugsweise 1 - 8 Gew.-%, in kosmetische Formulierungen eingearbeitet.

Weitere geeignete UV-Filter sind auch Methoxyflavone ensprechend der älteren Deutschen Patentanmeldung DE 10232595.2.

Organische UV-Filter werden in der Regel in einer Menge von 0,5 bis 20 Gew.-%, vorzugsweise 1 bis 15 Gew.-%, in kosmetische Formulierungen eingearbeitet.

Als anorganische UV-Filter sind solche aus der Gruppe der Titandioxide wie z.B. gecoatetes Titandioxid (z.B. Eusolex® T-2000, Eusolex®T-AQUA), Zinkoxide (z.B. Sachtotec®), Eisenoxide oder auch Ceroxide denkbar. Diese anorganischen UV-Filter werden in der Regel in einer Menge von 0,5 bis 20 Gew.-%, vorzugsweise 2 bis 10 Gew.-%, in kosmetische Zubereitungen eingearbeitet.

Werden verschiedene anorganische oder organische UV-Filter eingesetzt, so können diese in nahezu beliebigen Verhältnissen zueinander verwendet werden. Üblicherweise liegen die Verhältnisse der einzelnen Substanzen zueinander im Bereich 1:10 - 10:1, vorzugsweise im Bereich 1:5 - 5:1 und insbesondere bevorzugt im Bereich 1:2 - 2:1. Werden UV-A- und UV-B-Filter eingesetzt, so ist es für die meisten Anwendungen von Vorteil, wenn der Anteil an UV-B-Filtern überwiegt und das Verhältnis von UV-A-Filtern : UV-B-Filtern im Bereich 1:1 bis 1:10 liegt.

Bevorzugte Verbindungen mit UV-filternden Eigenschaften, die in der erfindungsgemäßen Zubereitung bevorzugt enthalten sein können, insbesondere wenn diese eine kosmetische Zubereitung ist, sind 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, 2-Phenyl-benzimidazol-5-sulfonsäure sowie ihre Kalium-, Natrium- und Triethanolaminsalze.

Durch Kombination von einer oder mehrerer Verbindungen der Formel I mit weiteren UV-Filtern kann die Schutzwirkung gegen schädliche Einwirkungen der UV-Strahlung optimiert werden.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter

Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Gegenstand der Erfindung ist daher auch eine Zubereitung, die dadurch gekennzeichnet ist, dass sie weiterhin einen oder mehrere UV-Filter enthält.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Pyrimidincarbonsäuren kommen in halophilen Mikroorganismen vor und spielen bei der Osmoregulation dieser Organismen eine Rolle (E. A. Galinski et al., Eur. J. Biochem., 149 (1985) Seite 135-139). Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden.

Dabei wird vorzugsweise eine Pyrimidincarbonsäure gemäß Formel

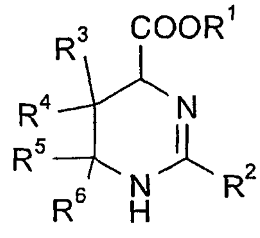

eingesetzt,
worin $R^1$ ein Rest H oder $C_{1-8}$-Alkyl, $R^2$ ein Rest H oder $C_{1-4}$-Alkyl und $R^3$, $R^4$, $R^5$ sowie $R^6$ jeweils unabhängig voneinander ein Rest aus der Gruppe H, OH, $NH_2$ und $C_{1-4}$-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidincarbonsäure) und Hydroxyectoin ((S, S)-1,4,5,6-Tetrahydro-5-hydroxy-2-methyl-4-pyrimidincarbonsäure) eingesetzt. Dabei enthalten die erfindungsgemäßen Zubereitungen derartige Pyrimidincarbonsäuren vorzugsweise in Mengen bis zu 15 Gew.-%. Vorzugsweise werden die Pyrimidincarbonsäuren dabei in Verhältnissen von 100:1 bis 1:100 zu den Verbindungen der Formel I eingesetzt, wobei

Verhältnisse im Bereich 1:10 bis 10:1 besonders bevorzugt sind.Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben der Verbindung der Formel I zusätzlich eine Aryloxim, vorzugsweise 2-Hydroxy-5-methyllaurophenonoxim enthalten, zeigen überraschende anti-inflammatorische Eignung. Dabei enthalten die Zubereitungen vorzugsweise 0,01 bis 10 Gew.-% Aryloxim, wobei es insbesondere bevorzugt ist, wenn die Zubereitung 0,05 bis 5 Gew.-% Aryloxim enthält.

Alle Verbindungen oder Komponenten, die in den Zubereitungen verwendet werden können, sind entweder bekannt und käuflich erwerbbar oder können nach bekannten Verfahren synthetisiert werden.

Die eine oder die mehreren Verbindungen der Formel I können in der üblichen Weise in kosmetische oder dermatologische Zubereitungen eingearbeitet werden. Geeignet sind Zubereitungen für eine äußerliche Anwendung, beispielsweise als Creme, Lotion, Gel, oder als Lösung, die auf die Haut aufgesprüht werden kann. Für eine innerliche Anwendung sind Darreichungsformeln wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer, Filmbildner und Feuchthaltemittel.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse

- Öle, wie Triglyceride der Caprin- oder der Caprylsäure, ferner natürliche Öle wie z. B. Rizinusöl;

- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fett-Ikoholen mit Alkansäuren niedriger C-Zahl oder mit Fettsäuren;

- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/ oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen der Formulierungs-Typ verwendet wird.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die Alkylglucoside werden ihrerseits vorteilhaft gewählt aus der Gruppe der Alkylglucoside, welche sich durch die Strukturformel

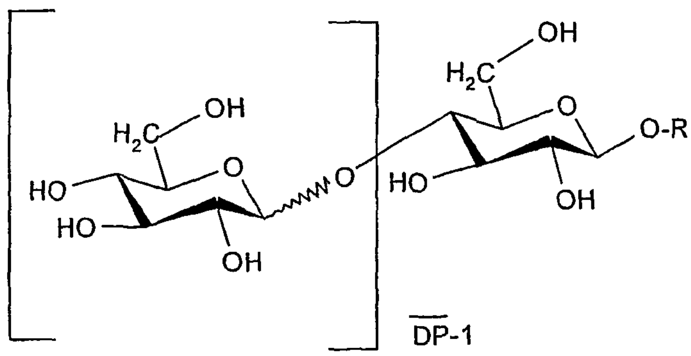

auszeichnen, wobei R einen verzweigten oder unverzweigten Alkylrest mit 4 bis 24 Kohlenstoffatomen darstellt und wobei $\overline{DP}$ einen mittleren Glucosylierungsgrad von bis zu 2 bedeutet.

Der Wert $\overline{DP}$ repräsentiert den Glucosidierungsgrad der erfindungsgemäß verwendeten Alkylglucoside und ist definiert als

$$\overline{DP} = \frac{p_1}{100} \cdot 1 + \frac{p_2}{100} \cdot 2 + \frac{p_3}{100} \cdot 3 + \ldots = \sum \frac{p_i}{100} \cdot i$$

Dabei stellen $p_1$, $p_2$, $p_3$ ... bzw. $p_i$ den Anteil der einfach, zweifach, dreifach ... i-fach glucosylierten Produkte in Gewichtsprozenten dar. Erfindungsgemäß vorteilhaft werden Produkte mit Glucosylierungsgraden von 1-2, insbesondere vorteilhaft von 1, 1 bis 1,5, ganz besonders vorteilhaft von 1,2 bis 1,4, insbesondere von 1,3 gewählt.

Der Wert DP trägt den Umstande Rechnung, dass Alkylglucoside herstellungsedingt in der Regel Gemische aus Mono- und Oligoglucosiden darstellen. Erfindungsgemäß vorteilhaft ist ein relativ hoher Gehalt an Monoglucosiden, typischerweise in der Größenordnung von 40-70 Gew.-%.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

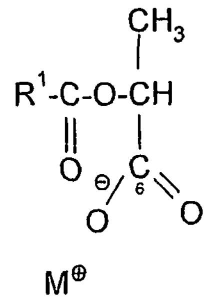

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Die Betaine werden vorteilhaft gewählt aus der Gruppe der Substanzen, welche sich durch die Strukturformel

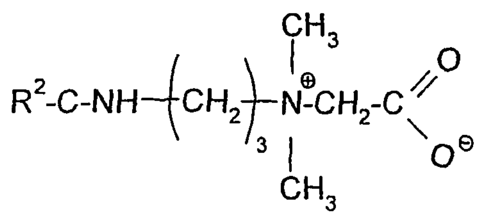

auszeichnen, wobei $R^2$ einen verzweigten oder unverzeigten Alkylrest mit 1 bis 30 Kohlenstoffatomen bedeutet.

Insbesondere vorteilhaft bedeutet $R^2$ einen verzweigten oder unverzweigten Alkylrest mit 6 bis 12 Kohlenstoffatomen.

Vorteilhaft ist beispielsweise Capramidopropylbetain, beispielsweise das Produkt Tego® Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zu Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in Wasser (O/W), eine multiple Emulsion, beispielsweise vom Typ Wasser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-Emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)-stearylether (Steareth-17),Polyethylenglycol(18) stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)-isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20) isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylenglycol(20)cetylether (Ceteth-20), Polyethylenglycol(13)-isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)-isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol (19)cetylstearylether (Ceteareth-19), Polyethylenglycol(20)cetylstearylether (Ceteareth-20).

Es ist ferner von Vorteil, die Fettsäureethoxylate aus folgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laurethl-4sulfat vorteilhaft verwendet werden. Als ethoxyliertes Cholesterinderivat kann vorteilhaft Polyethylenglycol(30)Cholesterylether verwendet werden. Auch Polyethylenglycol(25) Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrigalkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den Verbindungen der Formel I beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shampoonieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann außer der oder den Verbindungen der Formel I verschiedene, in diesem Mitteltyp verwendete Adjuvantien enthalten, wie Grenzflächen aktive Mittel, Verdikkungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Zum Schutz der Haut und/oder natürlicher oder sensibilisierter Haare vor Sonnenstrahlen wird auf die Haut oder die Haare eine kosmetische Zubereitung, enthaltend mindestens eine Verbindung der Formel I aufgetragen. Als sensibilisierte Haare werden dabei Haare verstanden, welche einer chemischen Behandlung, wie einer Dauerwellenbehandlung, einem Färbe- oder Entfärbeprozeß unterzogen worden sind.

Ferner wirken die Verbindungen der Formel I auch stabilisierend auf die Formulierung. Bei der Verwendung in entsprechenden Produkten bleiben diese daher auch länger stabil und verändern ihr Aussehen nicht. Insbesondere

bleibt auch bei längerdauernder Anwendung bzw. längerer Lagerung die Wirksamkeit der Inhaltsstoffe, z.B. Vitamine, erhalten. Dies ist besonders vorteilhaft bei der Zusammensetzungen zum Schutz der Haut gegen die Einwirkung von UV-Strahlen, da diese Kosmetika besonders hohen Belastungen durch die UV-Strahlung ausgesetzt sind.

Die vorteilhaften Eigenschaften der Verbindungen der Formel I können z.B. auch bei ihrer Verwendung in Nahrungsmitteln oder als Nahrungsergänzungsmittel oder als "functional food" ausgenutzt werden. Beispielsweise können die Verbindungen der Formel I die weiterhin in dem Nahrungsmittel, dem Nahrungsergänzungsmittel oder dem "functional food" enthaltenen Verbindungen oder auch den Organismus vor Oxidation bzw. vor der Einwirkung von Radikalen schützen.

Ein Gegenstand der Erfindung ist daher auch ein Nahrungsmittel, das mit mindestens einer Verbindung der Formel I angereichert ist.

Ein weiterer Gegenstand der Erfindung ist ein Nahrungsergänzungsmittel, das mindestens eine Verbindung der Formel I enthält. Nahrungsergänzungsmittel sind vorzugsweise Zubereitungen im Sinne der oben stehenden allgemeinen Definition und werden vorzugsweise oral verabreicht.

Die weiteren zu Nahrungsmitteln ausgeführten Erläuterungen gelten sinngemäß auch für Nahrungsergänzungsmittel und für "functional food". Die Nahrungsmittel, die nach der vorliegenden Erfindung mit mindestens einer Verbindung der Formel I angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren. Nahrungsmittel, die nach der vorliegenden Erfindung mit Verbindungen der Formel I angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensauce, Tomatenpüree, usw.. Weitere Beispiele für Nahrungsmittel, die nach der vorliegenden Erfindung mit Verbindungen der Formel I angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit Verbindungen der Formel I enthaltenden Extrakt angereichert zu werden, beispielsweise Multivitaminpräparate, Mineralstoffmischungen oder gezuckerter Saft. Als weitere Beispiele für Nahrungsmittel, die nach der vorliegenden Erfindung mit Verbindungen der Formel I angereichert werden können, seien Nahrungsmittelzubereitungen, beispielsweise zubereitete Zerealien, Gebäck, Mischgetränke, speziell für Kinder zubereitete Nahrungsmittel wie Joghurt, Diätnahrungsmittel, kalorienarme Nahrungsmittel oder Tierfutter genannt.

Die Nahrungsmittel, die nach der vorliegenden Erfindung mit Verbindungen der Formel I angereichert werden können, umfassen somit alle genießbaren Kombinationen von Kohlehydraten, Lipiden, Proteinen, anorganischen Elementen, Spurenelementen, Vitaminen, Wasser und aktiven Metaboliten von Pflanzen und Tieren.

Die Nahrungsmittel, die nach der vorliegenden Erfindung mit Verbindungen der Formel I angereichert werden können sowie die Nahrungsergänzungsmittel, die mindestens eine Verbindung der Formel I enthalten, werden vorzugsweise oral angewendet, z.B. in Form von Essen, Pillen, Tabletten, Kapseln, Pulver, Syrups, Lösungen oder Suspensionen.

Wie dargestellt können durch Verwendung/Einarbeitung von Verbindungen der Formel I in pharmazeutische und/oder kosmetische Zubereitungen, Nahrungsmittel und/oder Nahrungsergänzungsmittel wertvolle kosmetische Zubereitungen, pharmazeutische Zubereitungen, Nahrungsmittel und/oder Nahrungsergänzungsmittel hergestellt werden können. Gegenstand der Erfindung ist daher auch ausdrücklich die Verwendung der Verbindungen der Form I zur Herstellung einer kosmetischen Zubereitung, einer pharmazeutischen Zubereitung, eines Nahrungsmittels und/oder eines Nahrungsergänzungsmittels.

Soweit die erfindungsgemäße Zubereitung Verbindungen der Formel I enthält, ist/sind diese Verbindung/en, bezogen auf die gesamte Zubereitung, in folgenden Mengen enthalten:

- im Fall, dass die Zubereitung einer kosmetische und/oder pharmazeutische Formulierung ist, in einer Menge von 0.001 bis 100 Gew.-%, vorzugsweise in einer Menge von 0.01 bis 30 Gew.-%, besonders bevorzugt in einer Menge von 0.1 bis 10 Gew.-%

- im Fall, dass die Zubereitung ein Nahrungsmittel ist, in einer Menge von 0.00001 bis 20 Gew.-%, vorzugsweise in einer Menge von 0,001 bis 10 Gew.-%, und

- im Fall, dass die Zubereitung ein Nahrungsergänzungsmittel ist, vorzugsweise von 0.1 bis 80 Gew.-% bezogen auf das gesamte Nahrungsergänzungsmittel.

Die mit Verbindungen der Formel I angereicherten Nahrungsmittel können mit Hilfe von Techniken hergestellt werden, die dem Fachmann wohl bekannt sind.

Weitere Gegenstände der vorliegenden Erfindung sind ein Verfahren zur Herstellung einer Zubereitung, welches dadurch gekennzeichnet ist, dass mindestens eine Verbindung der Formel I mit Resten wie oben beschrieben mit einem kosmetisch oder dermatologisch oder für nahrungsmittel geeigneten Träger vermischt wird, und die Verwendung einer Verbindung der Formel I zur Herstellung einer Zubereitung mit antioxidativen Eigenschaften.

Die erfindungsgemäßen Zubereitungen können dabei mit Hilfe von Techniken hergestellt werden, die dem Fachmann wohl bekannt sind.

Das Vermischen kann ein Lösen, Emulgieren oder Dispergieren der Verbindung gemäß Formel I in dem Träger zur Folge haben.

Die in der erfindungsgemäßen Zubereitung enthaltenen Verbindungen der Formel I weisen, wie vorstehend beschrieben, sehr vorteilhafte Eigenschaften auf. Soweit die Verbindungen als $R^9$ verzweigtes oder gradkettiges $C_3$-$C_{10}$-Alkyl aufweisen, sind sie auch neu. Insbesondere bevorzugt sind dabei Verbindungen, in denen $R^9$ verzweigtes oder gradkettiges $C_3$-$C_{10}$-Alkyl ist. Gegenstand der Erfindung ist daher auch eine Verbindung der allgemeinen Formel I, worin $R^9$ verzweigtes oder gradkettiges $C_3$-$C_{10}$-Alkyl ist, vorzugsweise verzweigtes oder gradkettiges $C_3$-$C_6$-Alkyl, ist.

Bevorzugt sind dabei Verbindungen der Formel I, die dadurch gekennzeichnet sind, dass $R^1$, $R^3$, $R^4$, $R^5$, $R^6$, $R^7$, jeweils unabhängig voneinander H, verzweigtes oder gradkettiges $C_1$-$C_6$-Alkyl, vorzugsweise Methyl, Ethyl, n-Propyl, n-Butyl ist, und $R^2$ und $R^3$ unabhängig voneinander H oder OH ist.

Besonders bevorzugt sind Verbindungen der Formel I, die dadurch gekennzeichnet sind, dass $R^1$ bis $R^8$ jeweils H ist.

Ganz besonders bevorzugt sind Verbindungen der Formel I, wie vorstehend beschrieben, die dadurch gekennzeichnet sind, dass $R^9$ n-Propyl, Isopropyl, n-Butyl, sek.-Butyl oder tert.-Butyl, n-Pentyl, vorzugsweise n-Propyl oder n-Butyl, ist.

Die Verbindungen der Formel I können hergestellt werden indem eine Verbindung der allgemeinen Formel II

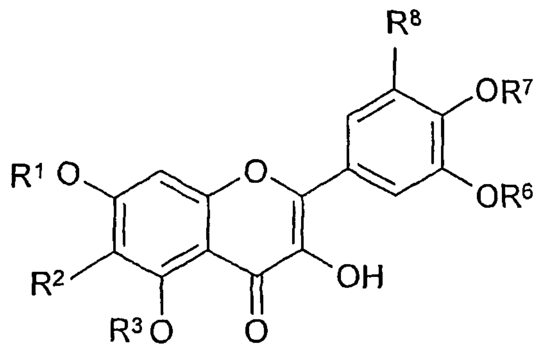

II

worin $R^1$, $R^2$, $R^3$, $R^6$, $R^7$, $R^8$ dieselben Bedeutungen haben wie in der allgemeinen Formel I
in einem einwertigen Alkohol $R^9$-OH, worin $R^9$ dieselben Bedeutungen hat wie in der allgemeinen Formel I, gelöst oder dispergiert wird,
gegebenenfalls ein Katalysator zugefügt wird,
die Verbindung der Formel II und der Alkohol $R^9$-OH unter Durchmischung und Sauerstoffzufuhr miteinander zur Reaktion gebracht werden
und das entstandene Produkt anschließend isoliert wird

Die Umsetzung der Verbindung der Formel II erfolgt vorzugsweise in Lösung des jeweiligen Alkohols und in Anwesenheit eines Katalysators wie z. B. Kupfer(II)chlorid. Die Durchmischung erfolgt bevorzugt durch Rühren. Die erforderlichen Reaktionszeit liegt in Abhängigkeit von den Substituenten der Formel II sowie dem jeweiligen Alkohol zwischen 3 bis 48 Stunden, typischerweise liegt sie zwischen 12 und 24 Stunden. Nach erfolgter Reaktion wird das Produkt wird je nach Kettenlänge des Alkohols durch Abdestillation, vorzugsweise bei Unterdruck, und gegebenenfalls Auskristallisieren mit einer Säure wie z. B. Salzsäure, vorzugsweise 10%ige (w/w) Salzsäure, oder durch Extraktion erhalten.

Ein weiterer Gegenstand der Erfindung ist ein Verfahren zur Herstellung der Verbindung der allgemeinen Formel I, das durch die oben beschriebenen Schritte gekennzeichnet ist.

Die INCI-Namen der verwendeten Rohstoffe sind wie folgt (die INCI-Namen werden definitionsgemäß in Englischer Sprache angegeben):

| Rohstoff | INCI-Name |
|---|---|
| Abil WE 09 | Polyglyceryl-4-Isostearate, Cetyl Dimethicone Copolyol, Hexyl Laurate |
| Antaron V-220 | PVP/Eicosene Copolymer |
| Arlacel 80 | Sorbitan Oleate |
| Arlacel 165 V | Glyceryl Stearate, PEG-100 Stearate |
| Avocadoöl | Persea Gratissima |
| Bienenwachs | Beeswax |
| Biobase™ EP | Glyceryl Stearate, Cetearyl Alcohol, Sodium Stearoyl Lactylate, Lecithin |
| Carbopol ETD 2050 | Carbomer |
| Cetiol V | Decyl Oleate |
| Cetylalkohol | Cetyl Alcohol |
| Cetylisononanoat | Cetyl Isononanoate |
| Cutina HR | Hydrogenated Castor Oil |
| Dimeticon | Dimethicone |
| Eusolex®232 | Phenylbenzimidazole Sulfonic Acid |
| Eusolex® 2292 | Octyl Methoxycinnamate, BHT |
| Eusolex® 6300 | 4-Methylbenzylidene Camphor |
| Eusolex 8300 | 4-Methylbenzylidene |
| Eusolex® 9020 | Butyl Methoxydibenzoylmethane |
| Eusolex®HMS | Homosalate |
| Eusolex T-Aqua | Aqua (Water), Titanium Dioxide, Alumina, Sodium Metaphosphate, Phenoxyethanol, Sodium Methylparaben |
| Eutanol G | Octyldodecanol |
| Germaben II | Propylene Glycol, Diazolidinyl Urea, Methylparaben, Propylparaben |
| Germaben II-E | Propylene Glycol, Diazolidinyl Urea, Methylparaben, Propylparaben |
| Glycerin | Glycerin |
| Glycerin (87%) | Glycerin |
| Glycerin (87% reinst) | Glycerin |
| Glycerin, wasserfrei | Glycerin |
| Hetester PHA | Propylene Glycol Isoceteth-3 Acetate |
| Hexyllaurat | Hexyl Laurate |
| Imwitor 960 K Schuppen | Glyceryl Stearate SE |
| Isolan PDI | Diisostearoyl Polyglyceryl-3-Diisostearat |
| Isopropylmyristat | Isopropyl Myristate |
| Isopropylpalmitat | Isopropyl Palmitate |
| Jojobaöl | Buxus Chinensis (Jojoba Oil) |
| Karion F flüssig | Sorbitol |
| Keltrol RD | Xanthan Gum |
| Magnesiumsulfat | Magnesium Sulfate |
| Magnesiumsulfat-Heptahydrat | Magnesium Sulfate |
| Methyl-4-hydroxybenzoat | Methylparaben |
| Miglyol 812 | Caprylic/Capric Triglyceride |
| Miglyol 812 N | Caprylic/Capric Triglyceride |
| Miglyol 812, Neutralöl | Caprylic/Capric Triglyceride |
| Mirasil CM5 | Cyclomethicone |
| Mirasil DM 350 | Dimethicone |
| Montanov 68 | Cetearyl Alcohol, Cetearyl Glucoside |
| Natriumchlorid | Sodium Chloride |
| Natronlauge, 10%ig | Sodium Hydroxide |
| Oxynex®K | PEG-8, Tocopherol, Ascorbyl Palmitate, Ascorbic Acid, Citric Acid |
| Panthenol-D | Panthenol |
| Paracera M | Microwax |

(fortgesetzt)

| Rohstoff | INCI-Name |
|---|---|
| Paraffinöl, fl. | Mineral Oil |
| Parfümöl TND-2417 | Parfum |
| Pemulen TR-1 | Acrylates/$C_{10-30}$ Alkyl Acrylate Crosspolymer |
| Pemulen® TR-2 | Acrylates/$C_{10-30}$ Alkyl Acrylate Crosspolymer |
| Performa® V 825 | Synthetic Wax |
| Polyglyceryl-2-Dipolyhydroxystearat | Polyglyceryl-2 Dipolyhydroxystearate |
| Prisorine 2021 | Isopropyl Isostearate |
| Propandiol-1,2 | Propylene Glycol |
| Propyl-4-hydroxybenzoat | Propylparaben |
| Rhodicare S | Xanthan Gum |
| RonaCare™ ASC III | Aqua, Lecithin, Dipalmitoyl Hydroxyproline, Phenoxyethanol, Tall Oil Sterol, Linoleic Acid, Tocopherol, Sodium Ascorbate, Mannitol, Methylparaben, Ethylparaben, Propylparaben, Butylparaben |
| RonaCare™ Bisabolol | Bisabolol |
| RonaCare™ Ectoin | Ectoin |
| RonaCare™ LPO | Lauryl p-Cresol Ketoxime |
| RonaCare™ Tocopherolacetat | Tocopheryl Acetate |
| Sepigel 305 | Polyacrylamide, $C_{13-14}$ Isoparaffin, Laureth-7 |
| SFE 839 | Cyclopentasiloxane, Dimethicone/ Vinyldimethicone Crosspolymer |
| Shea Butter | Shea Butter |
| Steareth-2 | Steareth-2 |
| Steareth-10 | Steareth-10 |
| Stearinsäure | Stearic Acid |
| DL-α-Tocopherolacetat | Tocopherol Acetate |
| Triethanolamin | Triethanolamine |
| Triethanolamin reinst | Triethanolamine |
| Wasser, demineralisiert | Aqua (Water) |
| Zinkstearat | Zinc Stearate |

Die Beispiele, ohne hierauf beschränkt zu sein, erläutern die Erfindung.

**Beispiel 1**

**Nachweis der antioxidativen Wirkung.**

Die antioxidative Wirkung der Verbindungen der Formel I wird am Beispiel von 2-Butoxy-2-(3,4-dihydroxy-phenyl)-3,3,5,7-tetrahydroxy-chroman-4-on im DPPH - Assay untersucht. Vergleichssubstanz ist Quercetin, das eine sehr hohe antioxidativen Aktivität aufweist.

2,2-Diphenyl-1-picrylhydrazyl(DPPH)-Assay: 2,2-Diphenyl-1-picrylhydrazyl ist ein in Lösung stabiles freies Radikal. Das ungepaarte Elektron führt zu einer starken Absorptionsbande bei 515 nm, die Lösung ist dunkel-violett gefärbt. In Gegenwart eines Radikalfängers wird das Elektron gepaart, die Absorption verschwindet und die Entfärbung verläuft stöchiometrisch unter Berücksichtigung der aufgenommenen Elektronen. Gemessen wird die Extinktion im Photometer. Zur Quantifizierung der antioxidativen Wirkung der Testsubstanzen wird der $EC_{50}$-Wert bestimmt, ermittelt als die Konzentration der Testsubstanz, bei der 50 % des eingesetzten 2,2-Diphenyl-1-picrylhydrazyls mit dieser reagiert haben. Je niedriger der $EC_{50}$-Wert ist, desto höher ist die Kapazität Radikale zu fangen.

Die Durchführung des DPPH-Assays erfolgt durch Umsetzung verschiedener Konzentrationen der Testsubstanzen in ethanolischer Lösung. Da die Testsubstanzen relativ langsam reagieren und die Gleichgewichtszustände sich erst relativ spät einstellen, werden zur Bestimmung der $EC_{50}$-Werte jeweils die (Extinktions)-Messwerte 600 Minuten nach Start der Reaktion zu Grunde gelegt. Die Bestimmung der $EC_{50}$-Werte erfolgt grafisch.

Für 2-Butoxy-2-(3,4-dihydroxy-phenyl)-3,3,5,7-tetrahydroxy-chroman-4-on ergibt sich ein $EC_{50}$-Wert von 0,08, für Quercetin ein $EC_{50}$-Wert von 0,089. Überraschenderweise weist das erfindungsgemäße oxidierte Flavonoid eine höhere antioxidative Aktivität auf als Quercetin, das als sehr starkes Antioxidans gilt.

**Beispiel 2**

**Darstellung von 2-Ethoxy-2-(3,4-dihydroxy-phenyl)-3,3,5,7-tetrahydroxy-chroman-4-on**

3,0 g Quercetin (9,9 mmol) wurden in 320 mL Ethanol gelöst und 0,7g Kupfer(II)chlorid (5,2 mmol) zugegeben. Die Reaktionslösung wurde 12 Stunden unter Sauerstoffeinfluss stark gerührt. Die Reaktionslösung wurde am Rotationsverdampfer bis zur Trockene eingeengt. Der schwarze ölige Rückstand wurde mit 70 mL Salzsäure (10 %-ig, w/w) versetzt. Der ausgefallene beigefarbene Feststoff wurde abgesaugt und im Vakuumtrockenschrank bei 40°C getrocknet.

Ausbeute: 2,6 g beigefarbenes Pulver; 73% der Theorie

$^{13}$C NMR Daten: (250MHz) in DMSO δ (ppm): 14, 39, 58, 90, 95, 99, 106, 114, 116, 120, 124, 143, 145, 158, 163, 166, 195

$^{1H}$NMR Daten: (250MHz) in DMSO δ (ppm): 0.9 (t, 3H), 3.15 (1H versteckt), 3.3 (1H versteckt), 5.95 (s, 2H), 6.26 (s, 1OH), 6.51 (s, 1OH), 6.73 (d, 1H), 6.88 (dd, 1H), 7.05 (d, 1H), 9.87 (d, 2OH), 10.76 (s, 1OH), 11.35 (s, 1OH)

Massenspektrum: EI (m/z): 346 ($M^+$-$H_2$0)

UV-Absorbtionsspektum

Konzentration: 1,2 mg/100mL Methanol

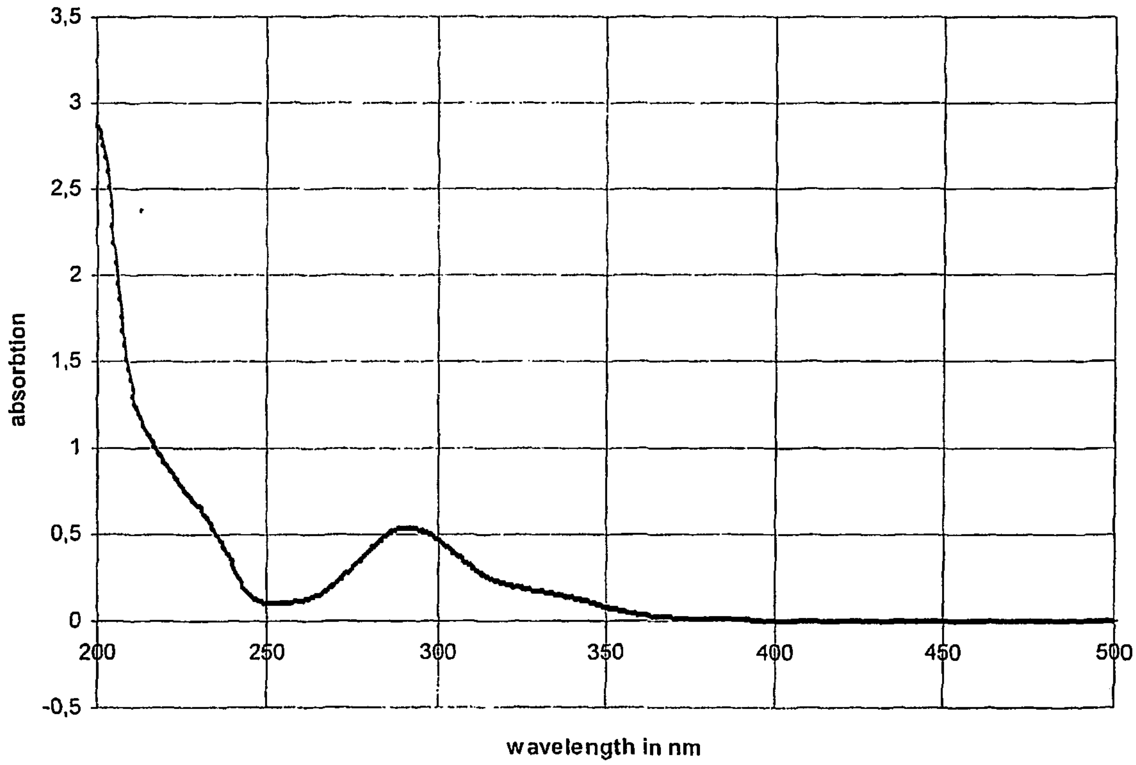


**Beispiel 3**

**Darstellung von 2-Butoxy-2-(3,4-dihydroxy-phenyl)-3,3,5,7-tetrahydroxy-chroman-4-one**

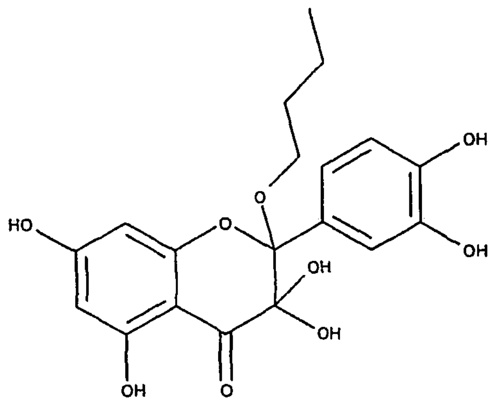

2,0 g Quercetin (6,6mmol) und 0,45 g Kupfer(II)chlorid (3,3mmol) wurden in 100 mL 1-Butanol suspendiert. Die Suspension wurde bei RT 1 Tag unter Sauerstoffeinfluss stark gerührt.

Die Reaktionslösung wurde am Rotationsverdampfer bis zur Trockene eingeengt. Der Destillationsrückstand wurde mit Salzsäure (10 %-ig, w/w) versetzt. Die wässrige Phase wurde mit Ethylacetat extrahiert und am Rotationsverdampfer bis zur Trockene eingeengt.

Der ölige Rückstand wurde säulenchromatographisch aufgereinigt.

Ausbeute: 150 mg gelber kristalliner Feststoff, 6% der Theorie

$^{1H}$NMR Daten: (250MHz) in DMSO δ (ppm): 0.65 (m, 3H), 1.05 (m, 2H), 1.25 (m, 2H), 3.1 (m, 1H), 3.25 (m, 1H), 5.95 (s, 1H), 5.96 (s, 1H), 6.25 (s, 1OH), 6.51 (s, 1OH), 6.72 (d, 1H), 6.88 (dd, 1H), 7.05 (d, 1H), 8.86 (s, 1OH) 8.89 (s, 1OH), 10.75 (s, 1OH) 11.35 (s, 1OH)

Massenspektrum: ESI (m/z): 391 $(M-H)^-$

UV-Absorbtionsspektum

Konzentration: 1,7 mg/100mL Methanol

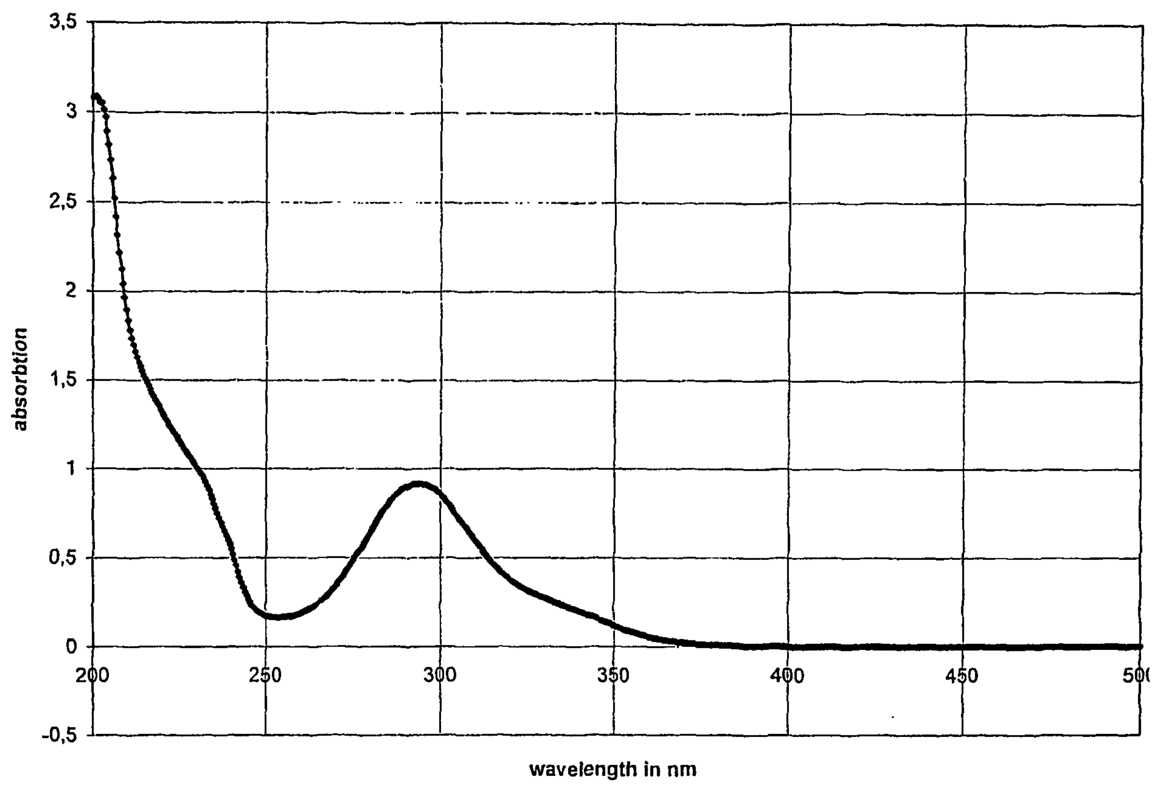

**Assays**

Bestimmung der antientzündlichen Aktivität

Keratinozyten-Monoschicht-$PGE_2$-Modell. Zur Induktion der Entzündung werden Keratinozyten 24 Stunden in 96-Well-Platten (15.000 Zellen/Well) mit der pro-inflammatorisch wirkender Substanz (zum Beispiel Phorbolmyristatacetat (PMA)) inkubiert. Die vorinkubierten Zellen (0,1 μg/ml Endkonzentration PMA) werden 24 Stunden mit $10^{-6}$ M Indometacin (Positivkontrolle) bzw. 0,2 mM Testsubstanz inkubiert. Als Negativkontrolle dient eine Kontrollkultur, die kein PMA enthält. Nach Inkubation wird in allen drei Proben der Gehalt an Prostaglandin $E_2$ ($PGE_2$) durch ELISA Kits bestimmtExpression des Tansglutaminase-Gens

**Beispiel 4**

**Lotion (W/O) zum Auftragen auf die Haut**

| | | Gew.-% |
|---|---|---|
| **A** | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | 2-Butoxy-2-(3,4-dihydroxy-phenyl)-3,3,5,7-tetrahydroxy-chroman-4-on | 0,5 |
| **B** | Glycerin | 5,0 |
| | Magnesiumsulfat-Heptahydrat | 1,0 |
| | Konservierungsmittel | q.s. |
| | Wasser, demineralisiert | ad 100 |

Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:
0,05 % Propyl-4-hydroxybenzoat
0,15 % Methyl-4-hydroxybenzoat

**Beispiel 5**

**Lotion (W/O) zum Auftragen auf die Haut**

| | | Gew.% |
|---|---|---|
| A | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |

(fortgesetzt)

| | | Gew.% |
|---|---|---|
| B | 2-Butoxy-2-(3,4-dihydroxy-phenyl)-3,3,5,7-tetrahydroxy-chroman-4-on | 1,0 |
| | Glycerin | 5,0 |
| | Magnesiumsulfat-Heptahydrat | 1,0 |
| | Konservierungsmittel | q.s. |
| | Wasser, demineralisiert | ad 100 |

Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:
0,05 % Propyl-4-hydroxybenzoat
0,15 % Methyl-4-hydroxybenzoat

**Beispiel 6**

**Lotion (W/O) zum Auftragen auf die Haut**

| | | Gew.% |
|---|---|---|
| A | 4,6,3',4'-Tetrahydroxybenzylcoumaranon-3 | 1,0 |
| | 2-Butoxy-2-(3,4-dihydroxy-phenyl)-3,3,5,7-tetrahydroxy-chroman-4-on | 1,0 |
| | Polyglyceryl-2-Dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| B | Glycerin | 5,0 |
| | Magnesiumsulfat-Heptahydrat | 1,0 |
| | Konservierungsmittel | q.s. |
| | Wasser, demineralisiert | ad 100 |

Herstellung

Phase A wird auf 75°C und Phase B auf 80°C erwärmt. Unter Rühren wird Phase B langsam zu Phase A gegeben. Nach dem Homogenisieren wird unter Rühren abgekühlt. Bei einer Temperatur von 40°C werden Parfümstoffe zugegeben.

Als Konservierungsmittel werden verwendet:
0,05 % Propyl-4-hydroxybenzoat
0,15 % Methyl-4-hydroxybenzoat

**Beispiel 7**

Aus folgenden Komponenten wird eine Creme (O/W), enthaltend Ectoin, hergestellt:

| | | | Gew.% |
|---|---|---|---|
| **A** | Paraffin, dünnflüssig | (1) | 8,0 |
| | Isopropylmyristat | (1) | 4,0 |
| | Mirasil CM5 | (2) | 3,0 |
| | Stearinsäure | (1) | 3,0 |
| | Arlacel 165 V | (3) | 5,0 |
| | 2-Butoxy-2-(3,4-dihydroxy-phenyl)-3,3,5,7-tetrahydroxy-chroman-4-on | | 1,0 |
| **B** | Glycerin (87%) | (1) | 3,0 |
| | Germaben II | (4) | 0,5 |
| | Wasser, demineralisiert | | ad 100 |
| **C** | RonaCare™ Ectoin | (1) | 1,0 |

Herstellung

Zunächst werden die Phasen A und B getrennt auf 75˚C erwärmt. Danach wird Phase A unter Rühren langsam zu Phase B gegeben und solange gerührt, bis eine homogene Mischung entsteht. Nach Homogenisierung der Emulsion wird unter Rühren auf 30˚C abgekühlt. Anschließend wird auf 35˚C erwärmt, die Phase C zugegeben und bis zur Homogenität gerührt.

Bezugsquellen

(1) Merck KGaA
(2) Rhodia
(3) Uniqema
(4) ISP

**Beispiel 8**

**Topische Zusammensetzung als W/O-Emulsion**

| | | | Gew.% |
|---|---|---|---|
| A | Isolan PDI | (2) | 3,0 |
| | Paraffinöl, fl. | (1) | 17,0 |
| | Isopropylmyristat | | 5,0 |
| | Bienenwachs | | 0,2 |
| | Cutina HR | (2) | 0,3 |
| | 2-Butoxy-2-(3,4-dihydroxy-phenyl)-3,3,5,7-tetrahydroxy-chroman-4-on | | 1,0 |
| B | Wasser, demineralisiert | | ad 100 |
| | Glycerin (87%) | | 4,0 |
| | Magnesiumsulfat | | 1,0 |
| | Germaben II-E | (3) | 1,0 |
| C | RonaCare™ LPO | (1) | 2,0 |

Herstellung

Die Phasen A und B werden auf 75°C erwärmt. Phase B wird unter Rühren zu Phase A gegeben. Anschließend wird das Gemisch bei 9000upm 2 Min. mit dem Turrax homogenisiert. Das erhaltene Gemisch wird auf 30 bis 35°C abgekühlt, und C wird eingerührt.

Bezugsquellen

(1) Merck KGaA
(2) Goldschmidt AG
(3) ISP

**Patentansprüche**

1. Zubereitung mit antioxidativen Eigenschaften, enthaltend mindestens eine Verbindung der allgemeinen Formel I

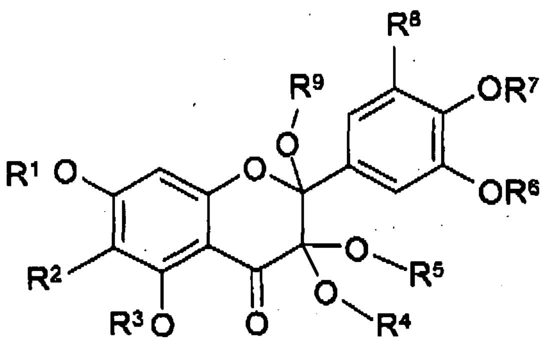

I

worin

$R^1$, $R^3$, $R^4$, $R^5$, $R^6$, $R^7$, $R^9$ jeweils unabhängig voneinander H oder Alkyl und
$R^2$, $R^8$, jeweils unabhängig voneinander H, OH oder -O-Alkyl ist,
Alkyl geradkettiges oder verzweigtes $C_1$-$C_{10}$-Alkyl bedeutet,

sowie gegebenenfalls Träger- und/oder Hilfsstoffe.

2. Zubereitung nach Anspruch 1, **dadurch gekennzeichnet, dass** $R^1$, $R^3$, $R^4$, $R^5$, $R^6$, $R^7$, $R^9$ jeweils unabhängig voneinander H, verzweigtes oder geradkettiges $C_1$-$C_6$-Alkyl und $R^2$ und $R^8$ unabhängig voneinander H oder OH ist.

3. Zubereitung nach Anspruch 2, **dadurch gekennzeichnet, dass** $C_1$-$C_6$-Alkyl Methyl, Ethyl, n-Propyl oder n-Butyl ist.

4. Zubereitung nach einem oder mehreren der Ansprüche 1 bis 3, **dadurch gekennzeichnet, dass** $R^4$ = $R^5$ ist.

5. Zubereitung nach Anspruch 4, **dadurch gekennzeichnet, dass** $R^1$ bis $R^8$ jeweils H ist und $R^8$ H oder $C_{1-6}$-Alkyl ist.

6. Zubereitung nach Anspruch 5, **dadurch gekennzeichnet, dass** $C_1$-$C_6$-Alkyl Methyl, Ethyl, n-Propyl oder n-Butyl ist.

7. Zubereitung nach einem oder mehreren der Ansprüche 1 bis 6, **dadurch gekennzeichnet, dass** die Zubereitung ein Arzneimittel ist.

8. Zubereitung nach einem oder mehreren der Ansprüche 1 bis 6, **dadurch gekennzeichnet, dass** die Zubereitung ein kosmetisches Mittel ist.

**9.** Zubereitung nach Anspruch 7 oder 8, **dadurch gekennzeichnet, dass** diese ein Hautbehandlungsmittel ist.

**10.** Zubereitung zur topischen Anwendung umfassend

a) die Verbindung nach einem oder mehreren der Ansprüche 1 bis 6,
b) einen hautverträglichen Träger, und
c) optional einen oder mehrere weitere Wirkstoffe mit hautpflegender und/oder entzündungshemmender Wirkung.

**11.** Zubereitung nach einem oder mehreren der Ansprüche 1 bis 10, **dadurch gekennzeichnet, dass** sie ein oder mehrere weitere/s Antioxidans/Antioxidantien enthält.

**12.** Zubereitung nach einem oder mehreren der Ansprüche 1 bis 11, **dadurch gekennzeichnet, dass** sie weiterhin einen oder mehrere UV-Filter enthält.

**13.** Nahrungsmittel, **dadurch gekennzeichnet, dass** dieses mit mindestens einer Verbindung der Formel I nach gemäß Anspruch 1 angereichert ist.

**14.** Nahrungsergänzungsmittel, **dadurch gekennzeichnet, dass** dieses mindestens eine Verbindung der Formel I gemäß Anspruch 1 enthält.

**15.** Verwendung der Verbindung nach einem oder mehreren der Ansprüche 1 bis 6 zur Herstellung einer pharmazeutischen und/oder kosmetischen Zubereitung zur Erhöhung der Widerstandskraft der Haut gegenüber Umwelteinflüssen, zur Vermeidung der Hautalterung, zur Verbesserung der Hautstruktur.

**16.** Verwendung gemäß Anspruch 15, **dadurch gekennzeichnet, dass** die Zubereitung zur Erhöhung der Widerstandskraft der Haut gegenüber Austrocknung und/oder zur Bildung glatter Haut ist.

**17.** Verwendung der Verbindung nach einem oder mehreren der Ansprüche 1 bis 6 zur Herstellung einer pharmazeutischen und/oder kosmetischen Zubereitung zum Schutz gegen oxidativen Stress sowie zur Bekämpfung von Allergien, Entzündungen und/oder Irritationen.

**18.** Verbindung der allgemeinen Formel I gemäß Anspruch 1
worin $R^9$ verzweigtes oder unverzweigtes $C_3$-$C_{10}$-Alkyl ist.

**19.** Verbindung gemäß Anspruch 18, **dadurch gekennzeichnet, dass** $R^9$ verzweigtes oder unverzweigtes $C_3$-$C_6$-Alkyl ist.

**20.** Verbindung nach Anspruch 19, **dadurch gekennzeichnet, dass** $R^1$, $R^3$, $R^4$, $R^5$, $R^6$, $R^7$, jeweils unabhängig voneinander H, verzweigtes oder unverzweigtes $C_1$-$C_6$-Alkyl, und $R^2$ und $R^8$ unabhängig voneinander H oder OH ist.

**21.** Verbindung nach Anspruch 20, **dadurch gekennzeichnet, dass** $C_1$-$C_8$-Alkyl Methyl, Ethyl, n-Propyl und/oder n-Butyl ist.

**22.** Verbindung nach Anspruch 21, worin $R^1$ bis $R^8$ jeweils H ist.

**23.** Verbindung nach einem oder mehreren der Ansprüche 15 bis 17, **dadurch gekennzeichnet, dass** $R^9$ n-Propyl, Isopropyl, n-Butyl, sek.-Butyl oder tert.-Butyl, n-Pentyl ist.

**24.** Verfahren zur Herstellung der Verbindung nach Anspruch 1, **dadurch gekennzeichnet, dass** eine Verbindung der allgemeinen Formel II

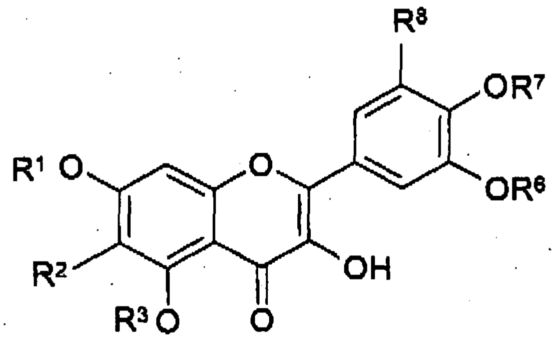

II

worin $R^1$, $R^2$, $R^3$, $R^6$, $R^7$, $R^8$ dieselben Bedeutungen haben wie in der allgemeinen Formel I in einem einwertigen Alkohol $R^9$-OH, worin $R^9$ dieselben Bedeutungen hat wie in der allgemeinen Formel I, gelöst oder dispergiert wird, gegebenenfalls ein Katalysator zugefügt wird,
die Verbindung der Formel II und der Alkohol $R^9$-OH unter Durchmischung und Sauerstoffzufuhr miteinander zur Reaktion gebracht werden,
und das entstandene Produkt anschließend isoliert wird.

## Claims

**1.** Composition having antioxidative properties, comprising at least one compound of the general formula I

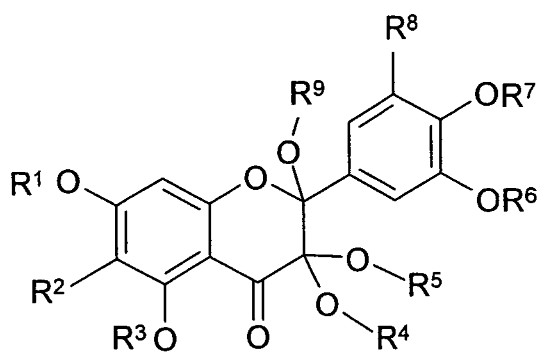

I

in which

$R^1$, $R^3$, $R^4$, $R^5$, $R^6$, $R^7$, $R^9$ are each, independently of one another, H or alkyl, and
$R^2$, $R^8$ are each, independently of one another, H, OH or -O-alkyl,
Alkyl denotes straight-chain or branched $C_1$-$C_{10}$-alkyl,

and optionally vehicles and/or assistants.

**2.** Composition according to Claim 1, **characterised in that** $R^1$, $R^3$, $R^4$, $R^5$, $R^6$, $R^7$, $R^9$ are each, independently of one another, H, branched or straight-chain $C_1$-$C_6$-alkyl and $R^2$ and $R^8$, independently of one another, are H or OH.

**3.** Composition according to Claim 2, **characterised in that** $C_1$-$C_6$-alkyl is methyl, ethyl, n-propyl or n-butyl.

**4.** Composition according to one or more of Claims 1 to 3, **characterised in that** $R^4$ = $R^5$.

**5.** Composition according to Claim 4, **characterised in that** $R^1$ to $R^8$ are each H and $R^9$ is H or $C_{1-6}$-alkyl.

**6.** Composition according to Claim 5, **characterised in that** $C_1$-$C_6$-alkyl is methyl, ethyl, n-propyl or n-butyl.

**7.** Composition according to one or more of Claims 1 to 6, **characterised in that** the composition is a medicament.

**8.** Composition according to one or more of Claims 1 to 6, **characterised in that** the composition is a cosmetic composition.

**9.** Composition according to Claim 7 or 8, **characterised in that** it is a skin-treatment composition.

**10.** Composition for topical use comprising

a) the compound according to one or more of Claims 1 to 6,
b) a skin-tolerated vehicle, and
c) optionally one or more further active compounds having a skin-care and/or inflammation-inhibiting action.

**11.** Composition according to one or more of Claims 1 to 10, **characterised in that** it comprises one or more further antioxidant(s).

**12.** Composition according to one or more of Claims 1 to 11, **characterised in that** it furthermore comprises one or more UV filters.

**13.** Food, **characterised in that** it is enriched with at least one compound of the formula I according to Claim 1.

**14.** Food supplement, **characterised in that** it comprises at least one compound of the formula I according to Claim 1.

**15.** Use of the compound according to one or more of Claims 1 to 6 for the preparation of a pharmaceutical and/or cosmetic composition for increasing the resistance of the skin to environmental influences, for preventing skin ageing, for improving the skin structure.

**16.** Use according to Claim 15, **characterised in that** the composition is for increasing the resistance of the skin to environmental drying-out and/or for the formation of smooth skin.

**17.** Use of the compound according to one or more of Claims 1 to 6 for the preparation of a pharmaceutical and/or cosmetic composition for protection against oxidative stress and for combating allergies, inflammation and/or irritation.

**18.** Compound of the general formula I according to Claim 1 in which $R^9$ is branched or unbranched $C_3$-$C_{10}$-alkyl.

**19.** Compound according to Claim 18, **characterised in that** $R^9$ is branched or unbranched $C_3$-$C_6$-alkyl.

**20.** Compound according to Claim 19, **characterised in that** $R^1$, $R^3$, $R^4$, $R^5$, $R^6$, $R^7$ are each, independently of one another, H, branched or unbranched $C_1$-$C_6$-alkyl, and $R^2$ and $R^8$, independently of one another, are H or OH.

**21.** Compound according to Claim 20, **characterised in that** $C_1$-$C_6$-alkyl is methyl, ethyl, n-propyl and/or n-butyl.

**22.** Compound according to Claim 21, in which $R^1$ to $R^8$ are each H.

**23.** Compound according to one or more of Claims 15 to 17, **characterised in that** $R^9$ is n-propyl, isopropyl, n-butyl, sec-butyl or tert-butyl, n-pentyl.

**24.** Process for the preparation of the compound according to Claim 1, **characterised in that** a compound of the general formula II

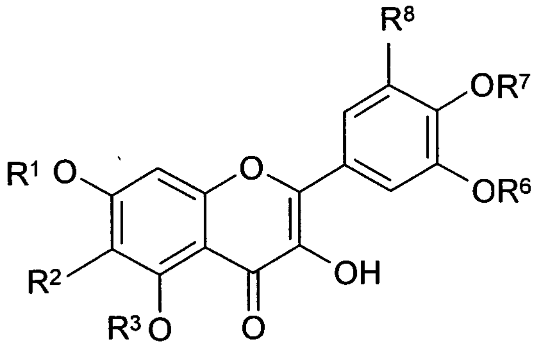

II

in which $R^1$, $R^2$, $R^3$, $R^6$, $R^7$, $R^8$ have the same meanings as in the general formula I,
is dissolved or dispersed in a monohydric alcohol $R^9$-OH, in which $R^9$ has the same meanings as in the general formula I,
a catalyst is optionally added,
the compound of the formula II and the alcohol $R^9$-OH are brought to reaction with one another with mixing and supply of oxygen,
and the resultant product is subsequently isolated.

## Revendications

**1.** Composition possédant des propriétés antioxydantes, comprenant au moins un composé de formule générale I

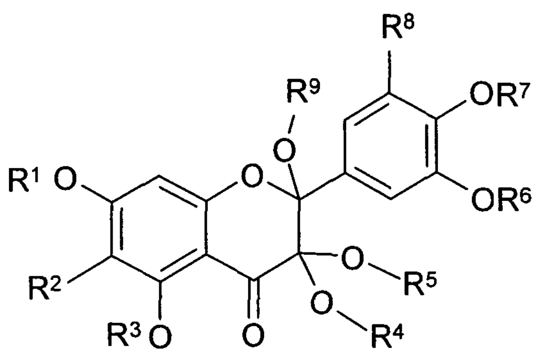

I

dans laquelle

$R^1$, $R^3$, $R^4$, $R^5$, $R^6$, $R^7$, $R^9$ sont chacun, indépendamment l'un de l'autre, H ou alkyle, et
$R^2$, $R^8$ sont chacun, indépendamment l'un de l'autre, H, OH ou -O-alkyle,
Alkyl désigne $C_1$-$C_{10}$-alkyle à chaîne linéaire ou ramifiée,

et éventuellement des véhicules et/ou des auxiliaires.

**2.** Composition selon la revendication 1, **caractérisée en ce que** $R^1$, $R^3$, $R^4$, $R^5$, $R^6$, $R^7$, $R^9$ sont chacun, indépendamment l'un de l'autre, H, $C_1$-$C_6$-alkyle à chaîne linéaire ou ramifiée et $R^2$ et $R^8$, indépendamment l'un de l'autre, sont H ou OH.

**3.** Composition selon la revendication 2, **caractérisée en ce que** $C_1$-$C_6$-alkyle est méthyle, éthyle, n-propyle ou n-butyle.

**4.** Composition selon l'une ou plusieurs des revendications 1 à 3, **caractérisée en ce que** $R^4 = R^5$

**5.** Composition selon la revendication 4, **caractérisée en ce que** $R^1$ à $R^8$ sont chacun H et $R^9$ est H ou $C_{1-6}$-alkyle.

**6.** Composition selon la revendication 5, **caractérisée en ce que** $C_1$-$C_6$-alkyle est méthyle, éthyle, n-propyle ou n-butyle.

**7.** Composition selon l'une ou plusieurs des revendications 1 à 6, **caractérisée en ce que** la composition est un médicament.

**8.** Composition selon l'une ou plusieurs des revendications 1 à 6, **caractérisée en ce que** la composition est une composition cosmétique.

**9.** Composition selon la revendication 7 ou 8, **caractérisée en ce qu'**il s'agit d'une composition de traitement de la peau.

**10.** Composition destinée à une utilisation topique, comprenant

a) le composé selon l'une ou plusieurs des revendications 1 à 6,
b) un véhicule toléré par la peau, et
c) éventuellement un ou plusieurs autres composés actifs ayant une action de soin de la peau et/ou d'inhibition des inflammations.

**11.** Composition selon l'une ou plusieurs des revendications 1 à 10, **caractérisée en ce qu'**elle comprend un ou plusieurs autres antioxydants.

**12.** Composition selon l'une ou plusieurs des revendications 1 à 11, **caractérisée en ce qu'**elle comprend en outre un ou plusieurs filtres UV.

**13.** Aliment, **caractérisé en ce qu'**il est enrichi avec au moins un composé de formule I selon la revendication 1.

**14.** Complément alimentaire, **caractérisé en ce qu'**il comprend au moins un composé de formule I selon la revendication 1.

**15.** Utilisation du composé selon l'une ou plusieurs des revendications 1 à 6, pour la préparation d'une composition pharmaceutique et/ou cosmétique pour augmenter la résistance de la peau vis-à-vis des influences environnementales, pour empêcher le vieillissement de la peau, pour améliorer la structure de la peau.

**16.** Utilisation selon la revendication 15, **caractérisée en ce que** la composition est destinée à augmenter la résistance de la peau vis-à-vis d'un dessèchement dû à l'environnement et/ou pour la formation d'une peau douce.

**17.** Utilisation du composé selon l'une ou plusieurs des revendications 1 à 6, pour la préparation d'une composition pharmaceutique et/ou cosmétique pour une protection contre le stress oxydatif et pour lutter contre les allergies, les inflammations et/ou les irritations.

**18.** Composé de formule générale I selon la revendication 1, dans lequel $R^9$ est $C_3$-$C_{10}$-alkyle ramifié ou non ramifié.

**19.** Composé selon la revendication 18, **caractérisé en ce que** $R^9$ est $C_3$-$C_6$-alkyle ramifié ou non ramifié.

**20.** Composé selon la revendication 19, **caractérisé en ce que** $R^1$, $R^3$, $R^4$, $R^5$, $R^6$, $R^7$ sont chacun, indépendamment l'un de l'autre, H, $C_1$-$C_6$-alkyle ramifié ou non ramifié, et $R^2$ et $R^8$, indépendamment l'un de l'autre, sont H ou OH.

**21.** Composé selon la revendication 20, **caractérisé en ce que** $C_1$-$C_6$-alkyle est méthyle, éthyle, n-propyle et/ou n-butyle.

**22.** Composé selon la revendication 21, dans lequel $R^1$ à $R^8$ sont chacun H.

**23.** Composé selon l'une ou plusieurs des revendications 15 à 17, **caractérisé en ce que** $R^9$ est n-propyle, isopropyle, n-butyle, sec-butyle ou tertio-butyle, n-pentyle.

**24.** Procédé de préparation du composé selon la revendication 1, **caractérisé en ce qu'**un composé de formule générale II

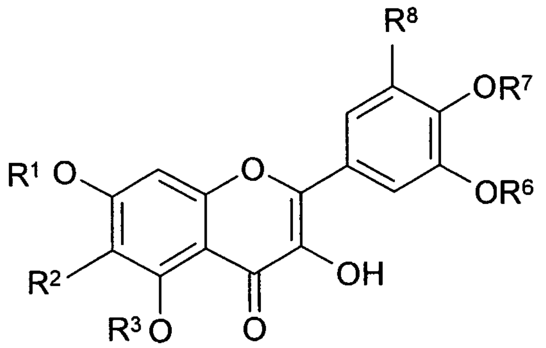

II

dans laquelle $R^1$, $R^2$, $R^3$, $R^6$, $R^7$, $R^8$ ont les mêmes significations que pour la formule générale I,
est solubilisé ou dispersé dans un alcool monohydrique $R^9$-OH, où $R^9$ a les mêmes significations que pour la formule générale I,
un catalyseur est éventuellement ajouté,
le composé de formule II et l'alcool $R^9$-OH sont amenés à réagir l'un avec l'autre sous agitation et apport d'oxygène,
et le produit résultant est ensuite isolé.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 3601414
- DE 10232595
- US 6242099 B1
- WO 0009652 A
- WO 0072806 A
- WO 0071084 A
- EP 0671161 A
- DE 4116123 A
- DE OS4308282 A


### In der Beschreibung aufgeführte Nicht-Patentliteratur


- **N.J. Miller ; G. Paganga.** *Trends in Plant Science,* 1997, vol. 2 (4), 152-159
- **K. Lemanska ; H. Szymusiak ; B. Tyrakowska ; R. Zielinski ; A.E.M.F. Soffers ; I.M.C.M. Rietjens.** *Free Radical Biology&Medicine,* 2001, vol. 31 (7), 869-881
- *Agric Biol. Chem.,* 1990, vol. 54 (8), 2143-2144
- **C. Sanchez-Moreno ; J.A. Larrauri ; F. Saura-Calixto.** *J. Sci. Food Agric.,* 1998, vol. 76 (2), 270-276
- **E. A. Galinski et al.** *Eur. J. Biochem.,* 1985, vol. 149, 135-139